# EUROPEAN PATENT APPLICATION

(11) **EP 2 218 444 A2**
(43) Date of publication of application: **18.08.2010**
(21) Application number: 10151462.8
(22) Date of filing: 22.01.2010
(51) Int. Cl.: A61K 9/20, A61K 31/135

(54) **Delayed release rasagiline formulation**

(30) Priority: 18.01.2010 US 689044; 09.06.2009 US 455976; 09.06.2009 US 456001; 09.06.2009 US 456029; 09.06.2009 US 456031; 23.01.2009 US 205833 P
(62) Divisional of application: 10166534.7
(71) Applicant: TEVA PHARMACEUTICAL INDUSTRIES, LTD., 49131 Petah Tiqva (IL)
(72) Inventor: Safadi, Muhammad, 16164, Nazareth (IL); Licht, Daniella, 54041, Givat-Shmuel (IL); Cohen, Rachel, 38100, Hadera (IL); Frenkel, Anton, 42268, Netanya (IL); Zholkovsky, Marina, 59496, Bat-Yam (IL); Koltai, Tamas, 42758, Netanya (IL)
(74) Representative: Nachshen, Neil Jacob

(57) **Abstract**

Disclosed are formulations of rasagiline base which are designed to delay release of rasagiline while maintaining specific pharmacokinetic properties. Also, disclosed are rasagiline citrate salt and the use and process of manufacture thereof.

## Description

### Background of the Invention

United States Patents 5,532,415, 5,387,612, 5,453,446, 5,457,133, 5,599,991, 5,744,500, 5,891,923, 5,668,181, 5,576,353, 5,519,061, 5,786,390, 6,316,504, 6,630,514 disclose R(+)-N-propargyl-1-aminoindan ("R-PAI"), also known as rasagiline. Rasagiline has been reported to be a selective inhibitor of the B-form of the enzyme monoamine oxidase ("MAO-B") and is useful in treating Parkinson's disease and various other conditions by inhibition of MAO-B in the brain.

United States Patent No. 6,126,968 discloses pharmaceutical formulations comprising rasagiline. A formulation of rasagiline mesylate is approved for treating Parkinson's disease either as monotherapy or as an adjunct with other treatments. See, e.g. AZILECT®, Physicians' Desk Reference 2009 (PRD, 63^{th} Edition).

AZILECT^{®} is a commercially available rasagiline mesylate immediate release formulation indicated for the treatment of the signs and symptoms of idiopathic Parkinson's disease as initial monotherapy and as adjunct therapy to levodopa. The current marketed formulation of rasagiline (Azilect®) is rapidly absorbed, reaching peak plasma concentration (tₘₐₓ) in approximately 1 hour. The absolute bioavailability of rasagiline is about 36%. (AZILECT^{®} Product Label, May 2006).

There are, however, several concerns associtated with the commercially available form of rasagiline mesylate. For example, a concern in using monoamine oxidase ("MAO") inhibitors is the risk of hypertensive crises, often called the "cheese effect." (Simpson, G.M. and White K. "Tyramine studies and the safety of MAOI drugs." J Clin Psychiatry. 1984 Jul; 45 (7 pt 2): 59-91.) This effect is caused by inhibition of peripheral MAO. A high concentration of peripheral MAO is found in the stomach.

Another concern in Parkinson's disease patients is that many patients suffer from delayed gastric emptying (Pfeiffer, R. F. and Quigley, E. M. M. "Gastrointestinal motility problems in patients with Parkinson's disease: Epidemiology, pathophysiology, and guidelines for management," CNS-Drugs, 1999, 11(6): 435-448; Jost, W. H., "Gastrointestinal motility problems in patients with Parkinson's disease: Effects of antiparkinsonian treatment and guidelines for management", Drugs and Aging, 1997, 10(4): 249-258). Delayed gastric emptying (prolonged gastric residence) can be a cause of increased inhibition of peripheral MAO, and can contribute to the cheese effect.

There is also a concern regarding the potential for formation of alkyl mesylates during the treatment of the free base of a drug substance with MSA if any residues of short-chain alcohols are present. (Snodin D., "Residues of genotoxic alkyl mesylates in mesylate salt drug substances: Real or imaginary problems?" Rugulatory Toxicology and Pharmacology, Vol. 45, 2006, pages 79-90).

Efforts to address such concerns and to improve the commercially available form of rasagiline mesylate are described in the literature. For example, PCT International Application Publication No. WO 2006/057912 describes orally disintegrating rasagiline compositions; PCT International Application Publication No. WO 2006/014973 discloses delayed release rasagiline compositions; PCT International Application Publication No. WO 2008/076348 discloses a crystalline solid form of the rasagiline base; PCT International Application Publication No. WO 2008/076315 discloses the tannate salt of rasagiline. Other efforts to make certain improvements are described in PCT International Application Publication No. WO 2008/019871 and in PCT International Application Publication No. WO 2008/131961.

However, the previous efforts did not disclose formulations as described herein, in particular formulations using solid rasagiline base described herein. The previous efforts also did not disclose the citrate salt of rasagiline or the advantages of formulations using the citrate salt of rasagiline, described herein.

### Summary of the Invention

The subject invention provides a stable oral dosage form comprising a core having a production process-resulting form of rasagiline and at least one pharmaceutically acceptable excipient; and an acid resistant pharmaceutically acceptable coating, the production process comprising
a) preparing the core by admixing rasagiline base, citric acid and/or malic acid, and a pharmaceutically acceptable excipient; and
b) coating the core with the acid resistant pharmaceutically acceptable coating.

The subject invention also provides a stable oral dosage form comprising a core having rasagiline base, rasagiline citrate, rasagiline malate, or a mixture of at least two of rasagiline base, rasagiline citrate, and rasagiline malate, and at least one pharmaceutically acceptable excipient; and an acid resistant pharmaceutically acceptable coating.

The subject invention further provides a method of treating a patient suffering from Parkinson's disease comprising administering to the patient the dosage form described herein.

The subject invention yet further provides rasagiline citrate.

The subject invention yet further provides a composition comprising the rasagiline citrate described herein and a carrier.

The subject invention yet further provides a process for manufacture of the rasagiline citrate or the composition describe herein, comprising:
a) combining a solution of citric acid with rasagiline base to form a first mixture;
b) adding a solvent to the first mixture to form a second mixture;
c) completely removing liquid from the second mixture; and
d) recovering the rasagiline citrate or preparing the composition.

The subject invention yet further provides a process for manufacture of the composition described herein, comprising:
a) obtaining rasagiline citrate in isolated form; and
b) admixing the rasagiline citrate with a carrier.

The subject invention yet further provides a method of treating a human subject afflicted with Parkinson's disease (PD), brain ischemia, stroke, head trauma injury, spinal trauma injury, neurotrauma, neurodegenerative disease, neurotoxic injury, nerve damage, dementia, Alzheimer's type dementia, senile dementia, depression, memory disorders, hyperactive syndrome, attention deficit disorder, Multiple Sclerosis (MS), schizophrenia, affective illness, Amyotrophic Lateral Sclerosis, Restless Legs Syndrome (RLS), hearing loss, Multiple System Atrophy (MSA), Glucoma, modifying Parkinson's disease, and Progressive Supranuclear Palsy (PSP), comprising administering to the human subject an amount of the rasagiline citrate or the composition described herein effective to treat the human subject.

### Detailed Description of the Invention

The subject invention provides a stable oral dosage form comprising a core having a production process-resulting form of rasagiline and at least one pharmaceutically acceptable excipient; and an acid resistant pharmaceutically acceptable coating, the production process comprising
a) preparing the core by admixing rasagiline base, citric acid and/or malic acid, and a pharmaceutically acceptable excipient; and
b) coating the core with the acid resistant pharmaceutically acceptable coating.

In yet another embodiment of the dosage form, step a) of the process comprises preparing a wet granulate of the rasagiline base, citric acid and/or malic acid, and a pharmaceutically acceptable excipient.

In yet another embodiment of the dosage form, step a) of the process further comprises:
i) drying the wet granulate to form a dry granulate,
ii) milling the dry granulate to form particles, and
iii) admixing the particles with at least one lubricant.

In yet another embodiment of the dosage form, in step iii) of the process the lubricant is talc or stearic acid, or a combination thereof.

In yet another embodiment of the dosage form, in step i) of the process the wet granulate is dried in a fluid bed dryer under inlet air temperature of 40°C to 50°C, and under outlet air temperature of not greater than 37°C.

In yet another embodiment of the dosage form, in step i) of the process the inlet air temperature is 45°C.

In yet another embodiment of the dosage form, in step ii) of the process the dry granulate is milled through an oscillating granulator.

In yet another embodiment of the dosage form, step a) of the process further comprises a step of forming the core by compression.

In yet another embodiment of the dosage form, in step a) of the process the core is prepared by admixing rasagiline base, citric acid, and a pharmaceutically acceptable excipient.

In yet another embodiment of the dosage form, in step a) of the process the core is prepared by admixing rasagiline base, malic acid, and a pharmaceutically acceptable excipient.

In yet another embodiment of the dosage form, in step a) of the process the core is prepared by admixing rasagiline base, citric acid and malic acid, and a pharmaceutically acceptable excipient.

The subject invention also provides a stable oral dosage form comprising a core having rasagiline base, rasagiline citrate, rasagiline malate, or a mixture of at least two of rasagiline base, rasagiline citrate, and rasagiline malate, and at least one pharmaceutically acceptable excipient; and an acid resistant pharmaceutically acceptable coating.

In an embodiment of the dosage form, the rasagiline base described herein is crystalline rasagiline base.

In an embodiment of the dosage form, the dosage form comprises a core having rasagiline citrate and at least one pharmaceutically acceptable excipient; and an acid resistant pharmaceutically acceptable coating.

In another embodiment of the dosage form, the dosage form comprises a core having rasagiline malate and at least one pharmaceutically acceptable excipient; and an acid resistant pharmaceutically acceptable coating.

In yet another embodiment of the dosage form, the dosage form when ingested by a human subject provides an AUC value of rasagiline of 80-130% of that of the corresponding amount of rasagiline ingested as an immediate release formulation.

In yet another embodiment of the dosage form described herein, the dosage form upon administration to a human subject provides an AUC value of rasagiline of 80-125% of that of the corresponding amount of rasagiline ingested as an immediate released formulation.

In yet another embodiment of the dosage form described herein, the dosage form when ingested by a human subject in a fed state provides an AUC value of rasagiline which is greater than that of the corresponding amount of rasagiline ingested as an immediate release formulation.

In yet another embodiment of the dosage form described herein, the dosage form when ingested by a human subject provides a Cₘₐₓ of rasagiline 80-145% of that of the corresponding amount of rasagiline ingested as an immediate release formulation.

In yet another embodiment of the dosage form described herein, the dosage form when ingested by a human subject provides a Cₘₐₓ of rasagiline of 80-125% of that of the corresponding dosage of rasagiline ingested as an immediate release formulation.

In yet another embodiment of the dosage form described herein, the dosage form when ingested by a human subject in a fed state provides a Cₘₐₓ of rasagiline which is greater than that of the corresponding amount of rasagiline ingested as an immediate release formulation.

In yet another embodiment of the dosage form, the core further comprises at least one anti-oxidant.

In yet another embodiment of the dosage form, the anti-oxidant is citric acid.

In yet another embodiment of the dosage form, the anti-oxidant is malic acid.

In yet another embodiment of the dosage form, the anti-oxidant is citric and malic acid.

In yet another embodiment of the dosage form described herein, the core is in the form of a tablet.

In yet another embodiment of the dosage form, the core further comprises at least one disintegrant.

In yet another embodiment of the dosage form, the disintegrant is present in the core at an amount between 0.5% and 20% by weight.

In yet another embodiment of the dosage form, the disintegrant is pre-gelatinized starch.

In yet another embodiment of the dosage form described herein, the acid resistant coating layer comprises methacrylic acid - ethyl acrylate copolymer (1:1) and a plasticizer.

In yet another embodiment of the dosage form, in the acid resistant coating layer the ratio of methacrylic acid - ethyl acrylate copolymer (1:1) to plasticizer is between 10 to 1 and 2 to 1.

In yet another embodiment of the dosage form, in the coating the ratio of methacrylic acid - ethyl acrylate copolymer (1:1) to plasticizer is about 5 to 1.

In yet another embodiment of the dosage form, the plasticizer is triethyl citrate.

In yet another embodiment of the dosage form, the acid resistant coating layer further comprises talc.

In yet another embodiment of the dosage form, the acid resistant coating is between 3% and 12% by weight of the dosage form.

In yet another embodiment of the dosage form, the acid resistant coating is about 8% by weight of the dosage form.

In yet another embodiment of the dosage form, the acid resistant coating comprises two coating layers.

In yet another embodiment of the dosage form, the inner one of the two coating layers comprises hypromellose.

In yet another embodiment of the dosage form, the dosage form is less than 150 mg by weight.

In yet another embodiment of the dosage form, the dosage form in addition to the rasagiline base and citric acid and/or malic acid, comprises mannitol, colloidal silicon dioxide, starch NF, pregelatinized starch, stearic acid, talc, hypromellose, methacrylic acid ethyl acrylate copolymer, talc extra fine, and triethyl citrate.

In yet another embodiment of the dosage form described herein, the content of rasagiline citrate is 0.74 mg to 3.63 mg.

In yet another embodiment of the dosage form, the dosage form in addition to the rasagiline citrate, comprises mannitol, colloidal silicon dioxide, starch NF, pregelatinized starch, stearic acid, talc, hypromellose, methacrylic acid ethyl acrylate copolymer, talc extra fine, and triethyl citrate.

In yet another embodiment of the dosage form described herein, the content of rasagiline malate is 0.66 mg to 3.05 mg.

In yet another embodiment of the dosage form, the dosage form in addition to the rasagiline malate comprises mannitol, colloidal silicon dioxide, starch NF, pregelatinized starch, stearic acid, talc, hypromellose, methacrylic acid ethyl acrylate copolymer, talc extra fine, and triethyl citrate.

In yet another embodiment of the dosage form described herein, the content of rasagiline is 1.0 mg.

In yet another embodiment of the dosage form, the dosage form comprises 79.8 mg of mannitol, 0.6 mg of colloidal silicon dioxide, 10.0 mg of starch NF, 20.0 mg of talc, 4.8 mg of hypromellose, 6.25 mg of methacrylic acid - ethyl acrylate copolymer, 1.25 mg of triethyl citrate, and 3.1 mg of talc extra fine.

In yet another embodiment of the dosage form, the dosage form comprises 67.8 mg of mannitol, 0.6 mg of aerosil, 10.0 mg of starch NF, 20.0 mg of pregelatinized starch, 2.0 mg of stearic acid, 2.0 mg of talc, 4.8 mg of hypromellose, 4.0 mg of methacrylic acid ethyl acrylate copolymer, 0.8 mg of triethyl citrate, and 1.9 mg of talc extra fine.

In yet another embodiment of the dosage form, the dosage form comprises 45.0 mg of mannitol, 0.4 mg of aerosil, 5.0 mg of starch NF, 20.0 mg of pregelatinized starch, 1.5 mg of stearic acid, 1.5 mg of talc, 3.5 mg of hypromellose, 4.0 mg of methacrylic acid ethyl acrylate copolymer, 0.8 mg of triethyl citrate, and 1.9 mg of talc extra fine.

In yet another embodiment of the dosage form described herein, the content of rasagiline is 0.5 mg.

In yet another embodiment of the dosage form, the dosage form comprises 80.3 mg of mannitol, 0.6 mg of aerosil, 10.0 mg of starch NF, 20.0 mg of pregelatinized starch, 2.0 mg of stearic acid, 2.0 mg of talc, 4.8 mg of hypromellose, 6.25 mg of methacrylic acid ethyl acrylate copolymer, 1.25 mg of triethyl citrate, and 3.1 mg of talc extra fine.

In yet another embodiment of the dosage form, the dosage form comprises 68.3 mg of mannitol, 0.6 mg of aerosil, 10.0 mg of starch NF, 20.0 mg of pregelatinized starch, 2.0 mg of stearic acid, 2.0 mg of talc, 4.8 mg of hypromellose, 4.0 mg of methacrylic acid ethyl acrylate copolymer, 0.8 mg of triethyl citrate, and 1.9 mg of talc extra fine.

In yet another embodiment of the dosage form, the dosage form comprises 45.5 mg of mannitol, 0.4 mg of aerosil, 5.0 mg of starch NF, 20.0 mg of pregelatinized starch, 1.5 mg of stearic acid, 1.5 mg of talc, 3.5 mg of hypromellose, 4.0 mg of methacrylic acid ethyl acrylate copolymer, 0.8 mg of triethyl citrate, and 1.9 mg of talc extra fine.

In yet another embodiment of the dosage form described herein, the dosage form further comprises 2.0 mg of a color coating agent.

In yet another embodiment of the dosage form, the dosage form comprises releases between 80 and 100% of rasagiline when placed in a basket apparatus in 500 mL of buffered aqueous media at a pH of 6.8 at 37°C at 75 revolutions per minute for 20 minutes.

In yet another embodiment of the dosage form, the total amount of non-polar impurities is less than 0.3 wt% relative to the amount of rasagiline.

In yet another embodiment of the dosage form, the amount of N-(2-chloroallyl)-1(R)-aminoindan in the dosage form is less than 20 ppm relative to the amount of rasagiline.

In yet another embodiment of the dosage form, the amount of N-(2-chloroallyl)-1(R)-aminoindan in the dosage form is less than 4 ppm relative to the amount of rasagiline.

In yet another embodiment of the dosage form, the dosage form comprises when ingested by a human subject achieves MAO-B inhibition substantially the same as that of the corresponding dosage of rasagiline ingested as an immediate release formulation.

The subject invention yet further provides rasagiline citrate.

In an embodiment of the rasagiline citrate, the rasagiline citrate is isolated rasagiline citrate or is substantially pure.

In another embodiment of the rasagiline citrate described herein, the rasagiline citrate is amorphous.

In yet another embodiment of the rasagiline citrate described herein, the rasagiline citrate is mono-rasagiline citrate.

In yet another embodiment of the rasagiline citrate described herein, the rasagiline content in the rasagiline citrate is between 42% and 52% by weight based on the total weight of the rasagiline citrate.

By a range between 42% and 52%, it is meant that all tenth and integer percentages within the range are specifically disclosed as part of the invention. Thus, 43%, 44%, ..., 50%, 51% and 42.1%, 42.2%, ..., 51.8%, 51.9% are included as embodiments of this invention.

In yet another embodiment of the rasagiline citrate described herein, the water content in the rasagiline citrate as determined by Karl Fischer analysis is less than 5%, less than 4%, less than 3%, less than 2%, or less than 1%.

The subject invention yet further provides a composition comprising the rasagiline citrate described herein and a carrier.

In an embodiment of the composition, the composition further comprises rasagiline base.

In another embodiment of the composition described herein, the rasagiline base is present in an amount of less than 5%, less than 4%, less than 3%, less than 2%, or less than 1%, based on the total rasagiline content of the composition.

In yet another embodiment of the composition described herein, the rasagiline base present in the composition is crystalline rasagiline base.

In yet another embodiment of the composition described herein, the composition is free of rasagiline base.

In yet another embodiment of the composition described herein, the rasagiline content present in the form of rasagiline citrate is more than 50%, 60%, 70%, 80%, 90%, 95%, 96%, 97%, 98%, or 99% of the total rasagiline content in the composition.

In yet another embodiment of the composition described herein, the composition is a pharmaceutical composition and the carrier is a pharmaceutically acceptable carrier.

In yet another embodiment of the composition described herein, the composition is in the form of an oral dosage form.

In yet another embodiment of the composition described herein, the composition is in the form of a tablet.

In yet another embodiment of the composition described herein, the composition further comprises stearic acid.

In yet another embodiment of the composition described herein, the composition is in the form of a transdermal patch.

In yet another embodiment of the composition described herein, the rasagiline citrate is mixed with a polymer.

The subject invention yet further provides a process for manufacture of the rasagiline citrate or the composition described herein, comprising:
a) combining a solution of citric acid with rasagiline base to form a first mixture;
b) adding a solvent to the first mixture to form a second mixture;
c) completely removing liquid from the second mixture; and
d) recovering the rasagiline citrate or preparing the composition.

In an embodiment of the process, the solvent added in step b) is acetone.

In another embodiment of the process described herein, in step c) the liquid is removed at ambient temperature and at reduced pressure.

The subject invention yet further provides a process for manufacture of the composition described herein, comprising:
a) obtaining rasagiline citrate in isolated form; and
b) admixing the rasagiline citrate with a carrier.

The subject invention yet further provides a method of treating a human subject afflicted with Parkinson's disease (PD), brain ischemia, stroke, head trauma injury, spinal trauma injury, neurotrauma, neurodegenerative disease, neurotoxic injury, nerve damage, dementia, Alzheimer's type dementia, senile dementia, depression, memory disorders, hyperactive syndrome, attention deficit disorder, Multiple Sclerosis (MS), schizophrenia, affective illness, Amyotrophic Lateral Sclerosis, Restless Legs Syndrome (RLS), hearing loss, Multiple System Atrophy (MSA), Glucoma, modifying Parkinson's disease, and Progressive Supranuclear Palsy (PSP), comprising administering to the human subject an amount of the dosage form described herein or the composition described herein effective to treat the human subject.

In an embodiment of the method, the subject suffers from delayed gastric emptying.

In another embodiment of the method, the administration step is to the human subject in a fed state.

Each of the embodiments described herein can be combined with any other embodiment disclosed herein.

By any range disclosed herein, it is meant that all hundredth, tenth and integer unit amounts within the range are specifically disclosed as part of the invention. Thus, for example, 0.01 mg to 50 mg means that 0.02, 0.03 ... 0.09; 0.1, 0.2 ... 0.9; and 1, 2 ... 49 mg unit amounts are included as embodiments of this invention.

As used herein, an example of an immediate release formulation of rasagiline is an AZILECT® Tablet containing rasagiline mesylate.

As used herein, a polymer is a large molecule composed of repeating structural units typically connected by covalent chemical bonds.

As used herein, a "pharmaceutically acceptable" carrier or excipient is one that is suitable for use with humans and/or animals without undue adverse side effects (such as toxicity, irritation, and allergic response) commensurate with a reasonable benefit/risk ratio.

As used herein, an "isolated" compound is a compound that has been separated from the crude reaction mixture in which it formed by an affirmative act of isolation. The act of isolation necessarily involves separating the compound from the other known components of the crude reaction mixture, with some impurities, unknown side products and residual amounts of the other known components of the crude reaction mixture permitted to remain. Purification is an example of an affirmative act of isolation.

As used herein, a composition that is "free" of a chemical entity means that the composition contains, if at all, an amount of the chemical entity which cannot be avoided following an affirmative act intended to separate the chemical entity and the composition.

As used herein, "about" in the context of a numerical value or range means ±10% of the numerical value or range recited or claimed.

Citric acid is a weak organic acid, and is triprotic. Therefore, the rasagiline citrate described herein may exist in mono-, di- or tri-rasagiline citrate form or a mixture thereof.

An immediate release formulation of rasagiline is AZILECT® Tablets which contain rasagiline (as the mesylate), a propargylamine-based drug indicated for the treatment of idiopathic Parkinson's disease. It is designated chemically as: 1H-Inden-1-amine, 2, 3-dihydro-N-2-propynyl-, (1R)-, methanesulfonate.

MAO inhibitors that selectively inhibit MAO-B are largely devoid of the potential to cause the "cheese effect". Nonetheless, the possibility exists that delayed gastric emptying of R-PAI may contribute to this phenomenon. Therefore, a goal in developing the formulations of the current invention was to develop a delayed release, enteric coated formulation comprising rasagiline in an amount equivalent to 1 mg of rasagiline base which would release the active ingredient in the duodenum and/or the jejunum, past the stomach.

During the development of the formulations of the current invention, it was determined that the formulations should meet the criteria of bioequivalence to the known, immediate release rasagiline mesylate formulations (as disclosed in example 1, for example) in a single dose bio-equivalence study in healthy subjects. These criteria include similarity of Cₘₐₓ and AUC₀₋ₜ (area under the curve) within the range of 80-125% within a 90% confidence interval between the new formulations and the known, immediate release formulations. The difference between the two formulations should be evident in bioequivalence studies as a difference in tₘₐₓ. In other words, the mean pharmacokinetic profile of the formulations of the current invention should substantially match the mean pharmacokinetic profile of the formulations of the known immediate release formulation, with the exception of the tₘₐₓ which should be greater for the delayed release formulation than for the immediate release formulation.

The reason for attempting to match the mean Cₘₐₓ and AUC₀₋ₜ of the known immediate release formulation (i.e. to formulate a delayed release formulation that is bioequivalent) is that the efficacy of the immediate release formulation has been proven, and it is likely that the efficacy of the formulation relates to its mean Cₘₐₓ and/ or AUC. (Arch Neurol. 2002; 59:1937-1943.)

In order to reach this target, development was directed toward enteric coated tablets having a quickly disintegrating core with an enteric coating which allows release of the rasagiline in a very specific range of pH. This specific pH range would prevent the formulation from releasing rasagiline in the stomach, and would allow the formulation to release rasagiline quickly under the physiological conditions of the intestine.

In PCT International Application Publication No. WO 2006/014973, delayed release rasagiline mesylate pharmaceutical formulations were disclosed. In the disclosed formulations (Example 1, 2 and 4) methacrylic acid - ethyl acrylate copolymer (1:1) 30% dispersion, known as Eudragit® L-30 D-55 was used. As evident from WO 2006/014973, these formulations were indeed delayed-release formulations as shown by their dissolution profiles and by the in-vivo data, however, their pharmacokinetic profile, in terms of mean Cₘₐₓ did not match the pharmacokinetic profile of the immediate release rasagiline mesylate formulations.

The excipient methacrylic acid - ethyl acrylate copolymer (1:1) 30% dispersion, known as Eudragit® L-30 D-55, used in the above-mentioned publication WO 2006/014973, when applied as an aqueous dispersion either on tablets or on spheres prevents dissolution of the coated composition at low acidic pH. The structure of this polymer is as follows:

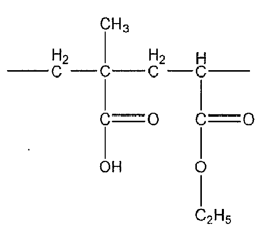

The ratio of the free carboxyl groups to the ester groups is approximately 1:1. The average molecular weight is approximately 250,000.

When this excipient is used in an aqueous dispersion or in an organic solution and formed into a film coating of a pharmaceutical formulation, it is intended to dissolve at a pH of about 5.5. (Aqueous Polymeric Coatings for Pharmaceutical Dosage Forms; Second Edition, Revised and Expanded. Ed. James W. McGinity, 1997.) Without wishing to be bound by any theory, it is possible that these prior art formulations began to dissolve in lower pH in the stomach, perhaps in the presence of food which can raise the pH in the stomach, and continued to dissolve over a prolonged period of time in the duodenum and the jejunum. The prolonged dissolution period could explain why the Cₘₐₓ of these prior art formulations was significantly lower than the Cₘₐₓ of the immediate release formulations to which they were compared.

In general, the release process encompasses three major steps:
1. Transport to the site where the pH is high enough to initiate release from the dosage form;
2. Dissolution of the coating; and
3. Disintegration and release of the drug from the core.

For highly soluble compounds the third step is the most crucial. In contrast, for enteric coated pellets for which emptying occurs gradually, not all at once, the first step has a major influence on the PK profile. As pellets empty at different times, they reach the second step at different time points as well. Therefore the PK profile is a superimposition of multiple "mini" PK profiles.

The delayed release compositions of the current invention are intended to withstand pH conditions of 6.0 and are intended to release the active ingredient only above that pH. This specific pH was chosen in order to attempt to minimize any possible dissolution of the pharmaceutical compositions of the invention in the stomach in fed condition and to allow rapid dissolution of the pharmaceutical compositions of the invention after the stomach in the duodenum and/or the jejunum. The ability of a pharmaceutical formulation to enter the duodenum before releasing rasagiline and subsequently releasing the rasagiline rapidly after the stomach provides a pharmacokinetic profile, and specifically a Cₘₐₓ and AUC₀₋ₜ, similar to that of the known immediate release formulation.

Achieving the goal of a delayed-release pharmaceutical formulation in which the Cₘₐₓ is similar to the corresponding immediate-release formulation is not trivial to achieve. In general, when delayed release formulations are compared to their immediate release counterparts in bio-studies, the Cₘₐₓ of the delayed release formulations are lower than the Cₘₐₓ in the corresponding immediate release formulations. (Mascher, et al. Arneimittelforschung. 2001; 51(6): 465-9. Behr, et al. J. Clin Pharmacol. 2002; 42(7): 791-7.)

In addition, the instant invention provides a solution to the problem of peripheral MAO inhibition by providing pharmaceutical dosage forms comprising rasagiline which are adapted to inhibit the release or absorption of rasagiline in the stomach (i.e. delay the release of rasagiline until at least a portion of the dosage form has traversed the stomach). This avoids or minimizes absorption of rasagiline in the stomach, thereby avoiding or minimizing the potential cheese effect.

The pharmaceutical dosage form may be comprised of an acid resistant excipient which prevents the dosage form or parts thereof from contacting the acidic environment of the stomach. The acid resistant excipient may coat the rasagiline in the form of an enteric coated tablet, capsule, hard or soft gelatin capsule. Enteric coating, in the context of this invention, is a coating which prevents the dissolution of an active ingredient in the stomach. This is determined by measuring the dissolution of the pharmaceutical dosage form in acidic solution, as defined by USP methods. Even in enteric pharmaceutical dosage forms, some of the dosage form may dissolve in the stomach; however, the dosage form may still be considered enteric according to USP standards.

In all of its aspects, the present invention provides pharmaceutical dosage forms useful for treating a condition selected from the group consisting of: Parkinson's disease (PD), brain ischemia, stroke, head trauma injury, spinal trauma injury, neurotrauma, neurodegenerative disease, neurotoxic injury, nerve damage, dementia, Alzheimer's type dementia, senile dementia, depression, memory disorders, hyperactive syndrome, attention deficit disorder, Multiple Sclerosis (MS), schizophrenia, affective illness, Amyotrophic Lateral Sclerosis, Restless Legs Syndrome (RLS), hearing loss, Multiple System Atrophy (MSA), Glucoma, modifying Parkinson's disease, and Progressive Supranuclear Palsy (PSP), but with a reduced risk of peripheral MAO inhibition that is typically associated with administration of rasagiline with known oral dosage forms.

Specific examples of pharmaceutically acceptable carriers and excipients that may be used to formulate oral dosage forms of the present invention are described, e.g., in U.S. Pat. No. 6,126,968 to Peskin et al., issued Oct. 3, 2000. Techniques and compositions for making dosage forms useful in the present invention are described, for example, in the following references: 7 Modern Pharmaceutics, Chapters 9 and 10 (Banker & Rhodes, Editors, 1979); Pharmaceutical Dosage Forms: Tablets (Lieberman et al., 1981); Ansel, Introduction to Pharmaceutical Dosage Forms 2nd Edition (1976); Remington's Pharmaceutical Sciences, 17th ed. (Mack Publishing Company, Easton, Pa., 1985); Advances in Pharmaceutical Sciences (David Ganderton, Trevor Jones, Eds., 1992); Advances in Pharmaceutical Sciences Vol 7. (David Ganderton, Trevor Jones, James McGinity, Eds., 1995); Aqueous Polymeric Coatings for Pharmaceutical Dosage Forms (Drugs and the Pharmaceutical Sciences, Series 36 (James McGinity, Ed., 1989); Pharmaceutical Particulate Carriers: Therapeutic Applications: Drugs and the Pharmaceutical Sciences, Vol 61 (Alain Rolland, Ed., 1993); Drug Delivery to the Gastrointestinal Tract (Ellis Horwood Books in the Biological Sciences. Series in Pharmaceutical Technology; J. G. Hardy, S. S. Davis, Clive G. Wilson, Eds.); Modern Pharmaceutics Drugs and the Pharmaceutical Sciences, Vol 40 (Gilbert S. Banker, Christopher T. Rhodes, Eds.).

The pharmaceutical dosage forms may be prepared as medicaments to be administered orally, parenterally, rectally or transdermally. Suitable forms for oral administration include tablets, compressed or coated pills, dragees, sachets, hard or soft gelatin capsules, sublingual tablets, syrups and suspensions; for parenteral administration the invention provides ampoules or vials that include an aqueous or non-aqueous solution or emulsion; for rectal administration the invention provides suppositories with hydrophilic or hydrophobic vehicles; for topical application as ointments; and for transdermal delivery the invention provides suitable delivery systems as known in the art.

Tablets may contain suitable binders, lubricants, disintegrating agents, coloring agents, flavoring agents, flow-inducing agents, melting agents, stabilizing agents, solubilizing agents, antioxidants, buffering agent, chelating agents, fillers and plasticizers. For instance, for oral administration in the dosage unit form of a tablet or capsule, the active drug component can be combined with an oral, non-toxic, pharmaceutically acceptable, inert carrier such as gelatin, agar, starch, methyl cellulose, dicalcium phosphate, calcium sulfate, mannitol, sorbitol, microcrystalline cellulose and the like. Suitable binders include starch, gelatin, natural sugars such as corn starch, natural and synthetic gums such as acacia, tragacanth, or sodium alginate, povidone, carboxymethylcellulose, polyethylene glycol, waxes, and the like. Antioxidants include ascorbic acid, fumaric acid, citric acid, malic acid, gallic acid and its salts and esters, butylated hydroxyanisole, editic acid. Lubricants used in these dosage forms include sodium oleate, sodium stearate, sodium benzoate, sodium acetate, stearic acid, sodium stearyl fumarate, talc and the like. Disintegrators include, without limitation, starch, methyl cellulose, agar, bentonite, xanthan gum, croscarmellose sodium, sodium starch glycolate and the like, suitable plasticizers include triacetin, triethyl citrate, dibutyl sebacate, polyethylene glycol and the like.

One type of oral dosage forms of the present invention relates to delayed release formulations. Such formulations may be comprised of an acid resistant excipient which prevents the dosage form or parts thereof from contacting the acidic environment of the stomach. The acid resistant excipient may coat the rasagiline in the form of an enteric coated tablet, capsule, or gelatin capsule. Enteric coating, in the context of this invention, is a coating which prevents the dissolution of an active ingredient in the stomach. Specific examples of pharmaceutically acceptable carriers and excipients that may be used to formulate such delayed release formulations are described, e.g., in International Application Publication No. WO 06/014973, hereby incorporated by reference in its entirety.

Another type of oral dosage forms of the present invention relates to fast disintegrating formulations which provide a means to avoid the absorption of rasagiline in the stomach, and to eliminate the need for swallowing tablets, by absorption of rasagiline into the body before reaching the stomach. Such absorption of rasagiline can be accomplished by contact with the buccal, sublingual, pharyngeal and/or esophageal mucous membranes. To accomplish this, the fast disintegrating formulations were designed to rapidly disperse within the mouth to allow maximum contact of rasagiline with the buccal, sublingual, pharyngeal and/or esophageal mucous membranes. Specific examples of pharmaceutically acceptable carriers and excipients that may be used to formulate such fast disintegrating formulations are described, e.g., in International Application Publication No. WO 03/051338, hereby incorporated by reference in its entirety.

Other pharmaceutical compositions of the present invention include transdermal patches. Transdermal patches are medicated adhesive patches placed on the skin to deliver a time-released dose of medication through the skin and into the bloodstream. A wide variety of pharmaceuticals can be delivered through transdermal patches. Some pharmaceuticals must be combined with other substances, for example alcohol, to increase their ability to penetrate the skin. Transdermal patches have several important components, including a liner to protect the patch during storage, the drug, adhesive, a membrane (to control release of the drug from the reservoir), and a backing to protect the patch from the outer environment. The two most common types of transdermal patches are matrix and reservoir types. (Wikipedia; and Remington, The Science and Practice of Pharmacy, 20th Edition, 2000)

In reservoir type patches, a drug is combined with a nonvolatile, inert liquid, such as mineral oil, whereas in matrix type patches a drug is dispersed in a lipophilic or hydrophilic polymer matrix such as acrylic or vinylic polymers. Adhesive polymers, such as polyisobutylene, are used to hold the patch in place on the skin. (Stanley Scheindlin, (2004) "Transdermal Drug Delivery: PAST, PRESENT, FUTURE," Molecular Interventions, 4:308-312)

The major limitation to transdermal drug-delivery is the intrinsic barrier property of the skin. Penetration enhancers are often added to transdermal drug formulations in order to disrupt the skin surface and cause faster drug delivery. Typical penetration enhancers include high-boiling alcohols, diols, fatty acid esters, oleic acid and glyceride-based solvents, and are commonly added at a concentration of one to 20 percent (w/w). (Melinda Hopp, "Developing Custom Adhesive Systems for Transdermal Drug Delivery Products," Drug Delivery)

The basket-type apparatus used in this invention is the apparatus 1 described in the United States Pharmacopeia, 29th Edition (2006), chapter 711. The apparatus is constructed as follows:

The assembly consists of the following: a covered vessel made of glass or other inert, transparent material; a motor; a metallic drive shaft; and a cylindrical basket. The vessel is partially immersed in a suitable water bath of any convenient size or placed in a heating jacket. The water bath or heating jacket permits holding the temperature inside the vessel at 37 ± 0.5 during the test and keeping the bath fluid in constant, smooth motion. No part of the assembly, including the environment in which the assembly is placed, contributes significant motion, agitation, or vibration beyond that due to the smoothly rotating stirring element. Apparatus that permits observation of the specimen and stirring element during the test is preferable. The vessel is cylindrical, with a hemispherical bottom and with one of the following dimensions and capacities: for a nominal capacity of 1 L, the height is 160 mm to 210 mm and its inside diameter is 98 mm to 106 mm; for a nominal capacity of 2 L, the height is 280 mm to 300 mm and its inside diameter is 98 mm to 106 mm; and for a nominal capacity of 4 L, the height is 280 mm to 300 mm and its inside diameter is 145 mm to 155 mm. Its sides are flanged at the top. A fitted cover may be used to retard evaporation. The shaft is positioned so that its axis is not more than 2 mm at any point from the vertical axis of the vessel and rotates smoothly and without significant wobble. A speed-regulating device is used that allows the shaft rotation speed to be selected and maintained at the rate specified in the individual monograph, within ±4%. Shaft and basket components of the stirring element are fabricated of stainless steel type 316 or equivalent.

Unless otherwise specified in the individual monograph, use 40-mesh cloth. A basket having a gold coating 0.0001 inch (2.5 µm) thick may be used. The dosage unit is placed in a dry basket at the beginning of each test. The distance between the inside bottom of the vessel and the basket is maintained at 25 ± 2 mm during the test.

Due to the sensitivity of rasagiline base to UV radiation and light in general, during the preparation of formulations described in the following examples, it is recommended to perform the process in a low UV radiation environment, preferably in an environment without any UV radiation.

The subject invention is also intended to include all isotopes of atoms occurring on the compounds disclosed herein. Isotopes include those atoms having the same atomic number but different mass numbers. By way of general example and without limitation, isotopes of hydrogen include tritium and deuterium. Isotopes of carbon include C-13 and C-14.

It will be noted that any notation of a carbon in structures throughout this application, when used without further notation, are intended to represent all isotopes of carbon, such as ¹²C , ¹³C, or ¹⁴C. Furthermore, any compounds containing ¹³C or ¹⁴C may specifically have the structure of any of the compounds disclosed herein.

It will also be noted that any notation of a hydrogen in structures throughout this application, when used without further notation, are intended to represent all isotopes of hydrogen, such as ¹H, ²H, or ³H. Furthermore, any compounds containing ²H or ³H may specifically have the structure of any of the compounds disclosed herein.

Isotopically-labeled compounds can generally be prepared by conventional techniques known to those skilled in the art or by processes analogous to those described in the Examples disclosed herein using an appropriate isotopically-labeled reagents in place of the non-labeled reagents employed.

This invention will be better understood from the experimental details which follow. However, one skilled in the art will readily appreciate that the specific methods and results discussed are merely illustrative of the invention as described more fully in the claims which follow thereafter.

### Experimental Details

### Example 1. Rasagiline Immediate Release Tablets

Rasagiline immediate release tablets were prepared using the ingredients listed in Table 1.

**Table 1**

| **Component** | **Function** | **Per Tablet (mg)** **(0.5mg Rasagiline base)** | **Per Tablet(mg)** **(1mg Rasagiline base)** |
|---|---|---|---|
| Rasagiline mesylate | | 0.78 | 1.56 |
| Mannitol | Filler | 79.62 | 159.24 |
| Aerosil | Flowing Agent | 0.6 | 1.2 |
| Starch NF | Binder | 10.0 | 20.0 |
| Starch, Pregelatinized (Starch STA-RX 1500) | Disintegrant | 10.0 | 20.0 |
| Talc | Lubricant | 2.0 | 4.0 |
| Stearic Acid | Lubricant | 2.0 | 4.0 |
| **Total core Tablet Weight** | | 105 | 210 |

Rasagiline mesylate, mannitol, half of the colloidal silicon dioxide, starch and pregelatinized starch were mixed in a Diosna P-800 mixer for about 5 minutes. Water was added and the mixture was mixed further. The granulate was dried and the remainder of the colloidal silicon dioxide was added. The granulate was ground in a Frewitt mill and stearic acid and talc were added. The granulate was mixed for five minutes in a tumbler and was tableted.

### Example 2. Rasagiline Base Tablet Cores

An attempt was made to formulate tablet cores which would have a pharmacokinetic profile (Cₘₐₓ and AUC) resembling that of the immediate release formulation of example 1.

A process for preparing crystalline rasagiline base is disclosed in U.S. Patent Application Publication No. 2008/0161408 (and which corresponds to WO 2008/076348). In particular, the document describes a process for manufacture of crystalline rasagiline base which comprises:
a) dissolving a salt of P(+)-N-propargyl-1-aminoindan in water to form a solution; b) cooling the solution to a temperature of about 0-15°C; c) basifying the solution to a pH of about 11 to form a suspension; and d) obtaining the crystalline rasagiline base from the suspension.

Five preliminary formulations of rasagiline base as API were prepared using standard tableting technique based on rasagiline immediate release formulation of Example 1. Different reagents were added in order to stabilize the API within the formulation.

**Table 2: Compositions of rasagiline base tablet cores:**

| Composition 1 | Composition 2 | Composition 3 | Composition 4 | Composition 5 |
|---|---|---|---|---|
| Rasagiline base | Rasagiline base | Rasagiline base | Rasagiline base | Rasagiline base |
| Citric Acid | Maleic Acid | Succinic Acid | Malic Acid | BHT |
| Mannitol USP/EP | Mannitol USP/EP | Mannitol USP/EP | Mannitol USP/EP | Mannitol USP/EP |
| Colloidal Silicon Dioxide | Colloidal Silicon Dioxide | Colloidal Silicon Dioxide | Colloidal Silicon Dioxide | Colloidal Silicon Dioxide |
| Pregelatinized Starch | Pregelatinized Starch | Pregelatinized Starch | Pregelatinized Starch | Pregelatinized Starch |
| Starch NF/EP | Starch NF/EP | Starch NF/EP | Starch NF/EP | Starch NF/EP |
| Stearic Acid | Stearic Acid | Stearic Acid | Stearic Acid | Stearic Acid |
| Talc | Talc | Talc | Talc | Talc |

Each composition was produced in lab scale batches of ∼500 tablets using laboratory equipment with non-GMP lot of API.

Stability results of all five formulations (final mixtures) were put on short-term stability studies at accelerated and room temperature conditions. Stability results, content of each formulation and dissolution results of tablets compressed using single punch are presented in the tables below.

**Composition 1**

| Mg/tab | Raw Materials | | Assay stability results (%) | | | |
|---|---|---|---|---|---|---|
| | | | Time 0 | 2 wks 25°C | 2 wks 40°C | 1mo 40°C |
| 0.82 | **Citric Acid** | | 101.6% | 94.2% | 94.8% | 98.0% |
| | Water | | | | | |
| 1.00 | Rasagiline base | | | | | |
| 80.0 | Manitol USP/EP | | | | | |
| 0.3 | Aerosil 200 | | Stability Results - Level of Impurity (%) | | | |
| 10.0 | Starch NF/EP | | Time 0 | 2 wks 25°C | 2 wks 40°C | 1mo 40°C |
| 20.0 | Starch STA-RX 1500 | Total Impurity | <0.04 (DL) | <0.04 (DL) | <0.1 (QL) | <0.2 (QL) |
| 0.3 | Aerosil 200 | | | | | |
| 2.0 | Stearic Acid | | | | | |
| 2.0 | Talc | | | | | |
| 116.42 | Total weight | | | | | |

**Composition 2**

| Mg/tab | Raw Materials | | Assay stability results (%) | | | |
|---|---|---|---|---|---|---|
| | | | Time 0 | 2 wks 25°C | 2 wks 40°C | 1mo 40°C |
| 0.7 | **Maleic Acid** | | 82.3 | 84.6 | 79.8 | 80.8 |
| | Water | | | | | |
| 1.00 | Rasagiline base | | | | | |
| 80.0 | Manitol USP/EP | | | | | |
| | | | Stability Results - Level of Impurity (%) | | | |
| 0.3 | Aerosil 200 | | Time 0 | 2 wks 25°C | 2 wks 40°C | 1mo 40°C |
| 10.0 | Starch NF/EP | Total Impurity | <0.1 (QL) | 0.1 | 0.4 | 0.8 |
| 20.0 | Starch STA-RX 1500 | | | | | |
| 0.3 | Aerosil 200 | | | | | |
| 2.0 | Stearic Acid | | | | | |
| 2.0 | Talc | | | | | |
| | | | | | | |
| 116.3 | Total weight | | | | | |

**Composition 3**

| Mg/ tab | Raw Materials | | Assay stability results (%) | | | |
|---|---|---|---|---|---|---|
| | | | Time 0 | 2 wks 25°C | 2 wks 40°C | 1mo 40°C |
| 0.7 | **Succinic Acid** | | 102.9 | 99.4 | 100.6 | 101.9 |
| | Water | | | | | |
| 1.00 | Rasagiline base | | Stability Results - Level of Impurity (%) | | | |
| 80.0 | Manitol USP/EP | | Time 0 | 2 wks 25°C | 2 wks 40°C | 1mo 40°C |
| 0.3 | Aerosil 200 | Total Impurity | 0.4 | 0.4 | 0.6 | 1.2 |
| 10.0 | Starch NF/EP | | | | | |
| 20.0 | Starch STA-RX 1500 | | | | | |
| 0.3 | Aerosil 200 | | | | | |
| 2.0 | Stearic Acid | | | | | |
| 2.0 | Talc | | | | | |
| 116.3 | Total weight | | | | | |

**Composition 4**

| Mg/tab | Raw Materials | | Assay stability results (%) | | | |
|---|---|---|---|---|---|---|
| | | | Time 0 | 2 wks 25°C | 2 wks 40°C | 1mo 40°C |
| 0.8 | **Malic Acid** | | 103.4 | 101.5 | 101.5 | 102.2 |
| | Water | | | | | |
| 1.00 | Rasagiline base | | | | | |
| 80.0 | Manitol USP/EP | | | | | |
| 0.3 | Aerosil 200 | | Stability Results - Level of Impurity (%) | | | |
| 10.0 | Starch NF/EP | | Time 0 | 2 wks 25°C | 2 wks 40°C | 1mo 40°C |
| 20.0 | Starch STA-RX 1500 | Total Impurity | <0.04 (DL) | <0.04 (DL) | <0.1 (QL) | <0.2 (QL) |
| 0.3 | Aerosil 200 | | | | | |
| 2.0 | Stearic Acid | | | | | |
| 2.0 | Talc | | | | | |
| 116.4 | Total weight | | | | | |

**Composition 5**

| Mg/ tab | Raw Materials | | Assay stability results (%) | | | |
|---|---|---|---|---|---|---|
| | | | Time 0 | 2 wks 25°C | 2 wks 40°C | 1mo 40°C |
| | Ethanol 95% | | 67.8 | 65.7 | 48.5 | 31.9 |
| 0.02 | **BHT** | | | | | |
| 1.00 | Rasagiline base | | Stability Results - Level of Impurity (%) | | | |
| 80.0 | Manitol USP/EP | | Time 0 | 2 wks 25°C | 2 wks 40°C | 1mo 40°C |
| 0.3 | Aerosil 200 | Total Impurity | < 0.1 (QL) | < 0.1 (QL) | 2.9 | 5.7 |
| 10.0 | Starch NF/EP | | | | | |
| 20.0 | Starch STA-RX 1500 | | | | | |
| 0.3 | Aerosil 200 | | | | | |
| 2.0 | Stearic Acid | | | | | |
| | | | | | | |
| 2.0 | Talc | | | | | |
| | | | | | | |
| | | | | | | |
| 115.62 | Total weight | | | | | |

**Dissolution Results (% in 0.1N HC1)**

| | 5 min | 10 min | 15 min |
|---|---|---|---|
| Composition 1 | 85 | 99 | 100 |
| Composition 2 | 49 | 82 | 90 |
| Composition 3 | 62 | 98 | 103 |
| Composition 4 | 59 | 100 | 107 |
| Composition 5 | 70 | 70 | 70 |

**Dissolution Results (% in Phosphate buffer pH 6.8)**

| | 5 min | 10 min | 15 min |
|---|---|---|---|
| Composition 1 | 78 | 92 | 94 |
| Composition 2 | 40 | 77 | 82 |
| Composition 3 | 59 | 98 | 101 |
| Composition 4 | 59 | 95 | 102 |
| Composition 5 | 70 | 70 | 70 |

### Discussion:

Compositions 1 and 4, which contain antioxidants Citric and Malic acids respectively, gave the best stability results and satisfactory dissolution profile. Therefore, they were chosen for future development.

### Example 3. Preparation of Delayed Release Enteric Coated Tablet with Citric Acid

In this example, rasagiline base delayed release enteric coated tablet containing citric acid was prepared.

Rasagiline citrate was identified as having formed in the tablet which was prepared as described in this example.

### Example 3a - 1.0 mg rasagiline base, 117 mg core tablet weight (Formulation I)

**Table 3a: Composition of rasagiline base delayed release enteric coated tablet**

| **Component** | **Function** | **Per Tablet** **(mg)** |
|---|---|---|
| Core tablets | | |
| Rasagiline Base | Drug Substance | 1.0 |
| Citric Acid | Antioxidant/Stabilizer | 1.6 |
| Mannitol | Filler | 79.84 |
| Colloidal Silicon Dioxide | Flowing Agent | 0.6 |
| Starch NF | Binder | 10.0 |
| Starch, Pregelatinized (STA-RX® 1500) | Disintegrant | 20.0 |
| Talc | Lubricant | 2.0 |
| Stearic Acid | Lubricant | 2.0 |
| **Total Core Tablet Weight** | | **117.0** |
| Subcoating | | |
| Pharmacoat® 606 (Hypromellose USP) Granules | Coating Agent | 4.8 |
| Purified Water | Processing Agent | |
| Coating Suspension | | |
| Eudragit® L-30D-55 | Coating Agent | 6.250* |
| Talc USP Extra Fine | Lubricant | 1.25 |
| Triethyl citrate | Plasticizer | 3.1 |
| Purified Water | Processing Agent | |
| **Total Tablet Weight** | | **132.4** |

| | | |
|---|---|---|
| * Dry substance remaining on the core. | | |

### I. Dry Mixing:

Mannitol, half amount of Aerosil, Pregelatinized Starch and Starch NF were placed in a high shear granulating mixer and were premixed for 1 minute mixing at mixer speed I, followed by 1 minute mixing at mixer speed I and chopper I.

### II. Wet Granulation:

Citric acid solution was prepared using 320 g of citric acid, in purified water in a weight ratio of approximately 1:10.6 to 1:6.

Rasagiline Base was added with stirring for approximately 15 minutes. The stirring was continued until a clear solution was observed. The solution was added into a high shear granulating mixer and the content was mixed for approximately 2 minutes at mixer speed II and chopper II. An extra amount of water was added into the high shear granulating mixer, and the solution was mixed for two more minutes at mixer speed II and chopper II.

The wet granulate was discharged into a fluid bed dryer trolley at mixer speed I.

### III. Fluid Bed Drying:

The material from step II was dried in a fluid bed dryer under inlet air temperature of 45°C (40° to 50°C) and outlet air temperature of maximum 37-38°C.

### IV. Milling:

The dry granulate and the residual amount of Aerosil were milled through an oscillating granulator with screen 0.6mm into a storage container.

The milled granulate was further weighed.

### V. Final Blending:

Stearic Acid and Talc were sieved through a 50 mesh screen and were transferred to the Y-cone/Bin.
1. The mixture was mixed for 5 minutes.
2. The final blend was obtained and the percentage yield was determined.
3. The final blend was stored in a container using an inner transparent polyethylene bag and an outer black polyethylene bag. Two Silica gel pillows were placed between the two polyethylene bags.
4. Samples were taken for a Blend Uniformity test.

### VI. Tablet Compression:

A tablet compression machine (FETTE 1200) was set up with the designated punches 6.0mm.

The in-process control testing for tablets included average weight, individual weight, thickness, hardness, friability and disintegration.

In process control specifications for Rasagiline Base DR 1mg tablets is:

| Parameter | Minimum | Target | Maximum |
|---|---|---|---|
| Average weight (mg) | 111 | 117 | 123 |
| Individual weight (mg) | 111 | 117 | 123 |
| Thickness (mm) | 3.3 | 3.6 | 3.9 |
| Hardness (SCU) | 7 | 9 | 11 |
| Friability (%) | -- | -- | 1.0 |
| Disintegration (minutes) | -- | -- | 5 |

The tablets were weighed and the percentage yield was calculated.

### VII. Sub-Coating:

Tablet cores were first coated with hypromellose (Pharmacoat 606®) as a pre-coating, followed by coating with Methacrylic Acid - Methyl Methacrylate Copolymer [1:1] (Eudragit® L-30D-55, 30% dispersion of Eudragit® L100-55) to prevent any possible interaction between the Rasagiline base in the core and the Eudragit L polymer.
1. Preparation of Pharmacoat 606® solution:
   Hypromellose USP solution was prepared using hypromellose, in purified water in a weight ratio of approximately 1:10.
2. Pre heating:
   The tablet cores were placed in an (Ohara) Coater coating pan. The tablets were heated under inlet air temperature of 50°C (45° to 55°C) and outlet air temperature of 45-50°C.
3. Spraying process:
   The tablet cores were sprayed with hypromellose solution in the Ohara Coater coating pan. The inlet air temperature was 50°C; the outlet air temperature was 35°C. The pan speed was set to 16 rpm (can vary from 14 to 18 rpm). Spraying rate was 15-35gr/min. The tablets were dried for 1 hour with inlet air temperature of 45°C (temperature range is 40°C -50°C).

### VIII. Enteric Coating:

1. Preparation of Enteric Coating dispersion of Eudragit® L100-55:
   Triethyl citrate was mixed with water for 15 minutes.
   The Talc Extra fine was added into the Triethyl citrate and water dispersion in an Ultraturax within 10 minutes. Eudragit L100-55 30% dispersion was added to Triethyl citrate/talc dispersion, filtered and stirred.
2. Pre heating:
   The precoated tablets were placed in an Ohara Coater coating pan. The tablets were heated under inlet air temperature of 50°C (45° to 55°C) and outlet air temperature of 45°C (40° to 50°C).
3. Spraying process:
   The tablets were sprayed with the dispersion in an Ohara coater pan. The inlet air temperature was in the range of 40°C -50 the outlet air temperature was in the range of 30-40°C. The pan speed was set to 16 rpm in range of 14-18 rpm, and the spraying rate was 5-20 gr/min. The tablets were dried for 2 hours. The inlet air temperature was 50°C on minimum pan speed.
      EUDRAGIT® L 100-55 contains an anionic copolymer based on methacrylic acid and ethyl acrylate. It is also known as methacrylic acid copolymer, type C. The ratio of the free carboxyl groups to the ester groups is approx. 1:1. The average molecular weight is approx. 250,000.

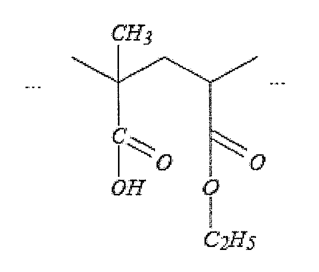

### Example 3b - 1.0 mg rasagiline base, 76 mg core tablet weight (Formulation III)

This formulation was prepared using similar steps as described in Example 3a.

**Table 3b: composition of rasagiline-base delayed release enteric coated tablet**

| **Component** | **Function** | **Per Tablet** **(mg)** |
|---|---|---|
| Core tablets | | |
| Rasagiline base | Drug Substance | 1.0 |
| Citric acid | Antioxidant/ Stabilizer | 1.6 |
| Mannitol | Filler | 45.0 |
| Aerosil | Flowing Agent | 0.4 |
| Starch NF | Binder | 5.0 |
| Starch, Pregelatinized (Starch STA-RX 1500) | Disintegrant | 20.0 |
| Talc | Lubricant | 1.5 |
| Stearic Acid | Lubricant | 1.5 |
| **Total Core Tablet Weight** | | **76.0** |
| Subcoating | | |
| Pharmacoat® 606 (Hypromellose USP) Granules | Coating Agent | 3.5 |
| Purified Water | Processing Agent | |
| Coating Suspension | | |
| Eudragit® L-30D-55 | Coating Agent | 4.0* |
| Talc USP Extra Fine | Lubricant | 1.9 |
| Triethyl citrate NF | Plasticizer | 0.8 |
| Purified Water | Processing Agent | |
| **Total Tablet Weight** | | **86.2** |

| | | |
|---|---|---|
| * Dry substance remaining on the core. | | |

### Example 3c - 0.5 mg rasagiline base, 117 mg core tablet weight

This formulation was prepared using similar steps as described in Example 3a.

Table 3c: composition of rasagiline-base delayed release enteric coated tablet

| **Component** | **Function** | **Per Tablet** **(mg)** |
|---|---|---|
| Core tablets | | |
| Rasagiline base | Drug Substance | 0.5 |
| Citric acid | Antioxidant/ Stabilizer | 1.6 |
| Mannitol | Filler | 80.34 |
| Aerosil | Flowing Agent | 0.6 |
| Starch NF | Binder | 10.0 |
| Starch, Pregelatinized (Starch STA-RX 1500) | Disintegrant | 20.0 |
| Talc | Lubricant | 2.0 |
| Stearic Acid | Lubricant | 2.0 |
| **Total Core Tablet** **Weight** | | **117.0** |
| Subcoating | | |
| Pharmacoat® 606 (Hypromellose USP) Granules | Coating Agent | 4.8 |
| Purified Water | Processing Agent | |
| Coating Suspension | | |
| Eudragit® L-30D-55 | Coating Agent | 6.25* |
| Talc USP Extra Fine | Lubricant | 3.1 |
| Triethyl citrate NF | Plasticizer | 1.25 |
| Purified Water | Processing Agent | |
| **Total Tablet weight** | | **132.4** |

| | | |
|---|---|---|
| * Dry substance remaining on the core. | | |

### Example 3d - 0.5 mg rasagiline base, 76 mg core tablet weight

This formulation was prepared using similar steps as described in Example 3a.

Table 3d: composition of rasagiline-base delayed release enteric coated tablet

| **Component** | **Function** | **Per Tablet** **(mg)** |
|---|---|---|
| Core tablets | | |
| Rasagiline base | Drug Substance | 0.5 |
| Citric acid | Antioxidant/ Stabilizer | 1.6 |
| Mannitol | Filler | 45.5 |
| Aerosil | Flowing Agent | 0.4 |
| Starch NF | Binder | 5.0 |
| Starch, Pregelatinized (Starch STA-RX 1500) | Disintegrant | 20.0 |
| Talc | Lubricant | 1.5 |
| Stearic Acid | Lubricant | 1.5 |
| **Total Core Tablet Weight** | | **76.0** |
| Subcoating | | |
| Pharmacoat® 606 (Hypromellose USP) Granules | Coating Agent | 3.5 |
| Purified Water | Processing Agent | |
| Coating Suspension | | |
| Eudragit® L-30D-55 | Coating Agent | 4.0* |
| Talc USP Extra Fine | Lubricant | 1.9 |
| Triethyl citrate NF | Plasticizer | 0.8 |
| Purified Water | Processing Agent | |
| **Total Tablet Weight** | | **86.2** |

| | | |
|---|---|---|
| * Dry substance remaining on the core. | | |

### Example 4. Dissolution Results of Tablets Prepared According to Example 3a

The tablets prepared according to Example 3a were tested for dissolution profile in various media according to USP procedures. The data below represents average for 4 tablets.

The %rasagiline released in the following tables is relative to a standard which is 1 mg rasagiline.

### Tablet Cores:

**Dissolution Profile (%rasagiline released) - 0.1N HCl, 75 rpm, 37°C**

| | 10 min | 20 min | 30 min | 45 min |
|---|---|---|---|---|
| 1 | 101 | 102 | 102 | 103 |
| 2 | 105 | 106 | 105 | 106 |
| 3 | 104 | 105 | 105 | 105 |
| 4 | 106 | 106 | 107 | 107 |
| %Mean | 104 | 105 | 105 | 105 |

**Dissolution Profile (%rasagiline released) - Phosphate buffer, 75 rpm, 37°C**

| | 10 min | 20 min | 30 min | 45 min |
|---|---|---|---|---|
| 1 | 98 | 99 | 99 | 99 |
| 2 | 100 | 101 | 101 | 102 |
| 3 | 99 | 100 | 100 | 101 |
| 4 | 96 | 96 | 97 | 97 |
| %Mean | 98 | 99 | 99 | 100 |
| %RSD | 1.9 | 2.0 | 2.0 | 2.2 |

### Sub-coated Tablets:

**Dissolution Profile (%rasagiline released) - 0.1N HCl, 75 rpm, 37°C**

| | 10 min | 20 min | 30 min | 45 min |
|---|---|---|---|---|
| 1 | 105 | 105 | 106 | 106 |
| 2 | 109 | 109 | 109 | 109 |
| 3 | 103 | 104 | 104 | 104 |
| 4 | 103 | 104 | 103 | 104 |
| %Mean | 105 | 105 | 105 | 106 |
| %RSD | 2.5 | 2.3 | 2.3 | 2.3 |

### Coated Tablets:

The dissolution profile of the coated tablets in 0.1N HCl was acceptable according to USP specification for delayed release (enteric coated) articles, 29th edition, Chapter 724, showing less than 10% release after 120 minutes.

**Dissolution Profile (%rasagiline released) - Phosphate buffer pH 5.8**

| | 10 min | 20 min | 30 min | 40 min | 60 min | 90 min |
|---|---|---|---|---|---|---|
| %Mean | 0 | 0 | | 0 | 0 | 0 |

**Dissolution Profile (%rasagiline released) - Phosphate buffer pH 6.4**

| | 10 min | 20 min | 30 min | 40 min | 60 min | 90 min |
|---|---|---|---|---|---|---|
| %Mean | 0 | 35 | 93 | 96 | 96 | 96 |
| %RSD | | | 2.2 | 1.3 | 1.3 | 1.2 |

**Dissolution Profile (%rasagiline released) - Phosphate buffer pH 6.8**

| | 10 min | 20 min | 30 min | 40 min | 60 min | 90 min |
|---|---|---|---|---|---|---|
| %Mean | 11 | 92 | 95 | 95 | 94 | 94 |
| %RSD | | 3.7 | 1.6 | 1.6 | 1.5 | 1.6 |

### Discussion:

The tablets prepared according to Example 3a do not begin the release of rasagiline at a pH lower than 6.0. At a pH of 6.8, there is a rapid release of rasagiline and within approximately 20 minutes, above 90% of the rasagiline is released from the formulation.

During the development of the formulations of the current invention, it was determined that the formulations should meet the criteria of bioequivalence to the known, immediate release rasagiline mesylate formulations (as disclosed in example 1) in a single dose bio-equivalence study in healthy subjects. These criteria include similarity of Cₘₐₓ and/or AUC₀₋ₜ (area under the curve) within the range of 80-125% within a 90% confidence interval between the new formulations and the known, immediate release formulations. The difference between the two formulations should be evident in bioequivalence studies as a difference in tₘₐₓ. In other words, the mean pharmacokinetic profile of the formulations of the current invention should match substantially the mean pharmacokinetic profile of the formulations of the immediate release formulation, with the exception of the tₘₐₓ which should be greater for the delayed release formulation than for the immediate release formulation.

The reason for attempting to match the mean Cₘₐₓ and AUC₀₋ₜ of the known immediate release formulation (i.e. to formulate a delayed release formulation that is bioequivalent) is that the efficacy of the immediate release formulation has been proven, and it is likely that the efficacy of the formulation relates to its mean Cₘₐₓ and/or AUC. (Arch Neurol. 2002; 59:1937-1943.)

In order to reach this target, development was directed toward delayed release enteric coated tablets having a quickly disintegrating core with an enteric coating which allows release of the rasagiline in a very specific range of pH. This specific pH range would prevent the formulation to release rasagiline in the stomach in fed condition, and would allow the formulation to release rasagiline quickly under the physiological conditions of the intestine after the stomach.

Although the tablets of Example 3a were coated with an enteric coating comprising Methacrylic Acid Ethyl Acrylate copolymer, as were the compositions in PCT application publication WO 2006/014973, the tablets according to Example 3a were capable of withstanding pH of 6.0 and below, whereas the composition in WO 2006/014973 were not.

The difference in dissolution profiles stems from the fact that the core's formulation contained high amount of disintegrant and the enteric film has a lower ratio of polymer to plasticizer is used in the compositions of the invention. The ratio of polymer to plasticizer between 10:1 and 2:1, and specifically 5:1, allows for enhanced in vitro dissolution profiles.

The dissolution profile of the formulation of Example 3a allows the composition to have enhanced pharmacokinetic properties, similar to the currently marketed immediate release formulations.

### Example 5. Stability Results of Tablets Prepared According to Example 3a

Stability of enteric coated tablets produced using formulations containing citric acid was tested under different storage conditions. The results are summarized below.

### Stability results (Accelerated Conditions):

The dissolution profile of the enteric coated tablets in 0.1N HCl was acceptable according to USP specification for delayed release (enteric coated) articles, 29th edition, Chapter 724, showing less than 10% release after 120 minutes.

The following table shows that dissolution profile for enteric coated tablets after different period of storage.

**Dissolution Profile of Coated Tablets - Phosphate buffer pH 6.8, 37°C**

| | Dissolution Profile after Different Period of Storage (%rasagiline released) | | | | | |
|---|---|---|---|---|---|---|
| Storage Period (months) | 10 min | 20 min | 30 min | 40 min | 60 min | 90 min |
| 0 | 11 | 92 | 95 | 95 | 96 | 96 |
| 1 | 28 | 95 | 96 | 96 | 97 | 97 |
| 2 | 12 | 97 | 98 | 98 | 98 | 99 |
| 3 | 35 | 101 | 103 | 103 | 104 | 104 |

The %rasagiline released in the above table is relative to a standard which is 1 mg rasagiline.

The following tables show that analytical results for different batches of the enteric coated tablets under various storage conditions.

**Coated tablets - Batch 1**

| **Conditions** | | **Assay %** | **Total Impurities (%)** |
|---|---|---|---|
| **T=0** | | 101.5 | <DL |
| 40°C, 75RH | **1Mo** | 101.1 | <DL |
| | **2Mo** | 105.4 | 0.3% |
| | **3Mo** | 104.5 | 0.4% |
| | **4Mo** | 100.9 | 0.4% |
| 25°C, 60RH | **1Mo** | 104.7 | <DL |
| | **3Mo** | 106.2 | <DL |

**Coated tablets - Batch 2**

| **Conditions** | | **Assay %** | **Total Impurities (%)** |
|---|---|---|---|
| **T=0** | | 98.6 | <DL |
| 40°C, 75RH | **1Mo** | 99.1 | 0.05% |
| | **2Mo** | 96.3 | 0.1% |
| | **3Mo** | 95.6 | 0.2% |
| | **4Mo** | 96.6 | 0.3% |
| 30°C, 65RH | **1Mo** | 99.8 | <DL |
| | **2Mo** | 98.4 | <DL |
| | **3Mo** | 96.5 | <DL |
| 25°C, 60RH | **1Mo** | 98.4 | <DL |
| | **2Mo** | 95.8 | <DL |
| | **3Mo** | 96.2 | <DL |

**Coated tablets - Batch 3**

| **Conditions** | | **Assay %** | **Total Impurities (%)** |
|---|---|---|---|
| **T=0** | | 100.3 | <DL |
| 40°C, 75RH | **1Mo** | 100.3 | <DL |
| 40°C, 75RH | **2Mo** | 102.0 | <DL |
| 40°C, 75RH | **3Mo** | | <0.28 |
| 30°C, 65RH | **3Mo** | | <0.08 |
| 25°C, 60RH | **1Mo** | 101.2 | <DL |
| 25°C, 60RH | **2Mo** | 102.1 | <DL |
| 25°C, 60RH | **3Mo** | | <0.08 |

**N-(2-Chloroallyl)-1(R)-aminoindan (2-Cl-AAI) Impurities**

| Batch No. | 2-Cl-AAI Content, % |
|---|---|
| 1 | LT 0.00004 |
| 2 | LT 0.00004 |

### Example 6. Preparation of Rasagiline Base Delayed Release Enteric Coated Tablets with Malic Acid

### Example 6a - 1 mg rasagiline base, 117 mg core tablet weight

**Table 6a: composition of rasagiline-base delayed release enteric coated tablet**

| **Component** | **Function** | **Per Tablet** **(mg )** |
|---|---|---|
| Core tablets | | |
| Rasagiline base | Drug Substance | 1.0 |
| Malic acid | Antioxidant/ Stabilizer | 1.6 |
| Mannitol | Filler | 80.0 |
| Aerosil | Flowing Agent | 0.6 |
| Starch NF | Binder | 10.0 |
| Starch, Pregelatinized (Starch STA-RX® 1500) | Disintegrant | 20.0 |
| Talc | Lubricant | 2.0 |
| Stearic Acid | Lubricant | 2.0 |
| **Total Core Tablet Weight** | | **117.2** |
| Subcoating | | |
| Pharmacoat® 606 (Hypromellose USP) Granules | Coating Agent | 4.8 |
| Purified Water | Processing Agent | |
| Coating Suspention | | |
| Eudragit® L-30D-55 | Coating Agent | 6.25* |
| Talc USP Extra Fine | Lubricant | 3.1 |
| Triethyl citrate NF | Plasticizer | 1.25 |
| Purified Water | Processing Agent | |
| **Total Tablet Weight** | | **132.6** |

| | | |
|---|---|---|
| * Dry substance remaining on the core. | | |

### I. Dry Mixing:

Mannitol, half amount of Aerosil, Starch Pregelatinized and starch NF are placed into a high shear granulating mixer and are premixed for 1 minute mixing at mixer speed I, followed by 1 minute mixing at mixer speed II and chopper II.

### II. Wet Granulation:

Malic acid solution was prepared using malic acid in purified water in the ratio of approximately 1:10.6 to 1:6.

Rasagiline Base was added with stirring for approximately 15 minutes. The stirring was continued until a clear solution was observed.

The solution was added into a high shear granulating mixer and was mixed for approximately 2 minute mixing at mixer speed II and chopper II. An extra amount of water was added into the high shear granulating mixer, and the solution was mixed for two more minutes at mixer speed II and chopper II.

The wet granulate was discharged to a fluid bed dryer trolley at mixer speed I.

### III. Fluid Bed Drying:

The material was dried in a fluid bed dryer under inlet air temperature of 45°C (40° to 50°C) and outlet air temperature of maximum 37-38°C.

### IV. Milling:

The dry granulate was milled with the residual amount of Aerosil through an oscillating granulator with screen 0.6mm into storage container.

The milled granulate is weighed.

### V. Final Blending:

1. Stearic Acid and Talc were sieved through a 50 mesh screen and transferred to the Y-cone or Bin.
2. The mixture was mixed for 5 minutes.
3. The final blend was stored in a container using an inner transparent polyethylene bag and an outer black polyethylene bag. Two Silica gel pillows were placed between the two polyethylene bags.
4. Samples were taken for a Blend Uniformity test.

### VI. Tablet Compression:

The compressing tablet machine was set up with the designated punches 6.0mm. The diameter of the punch may change +/-10%.

The in-process control testing for tablets includes average weight, individual weight, thickness, hardness, friability and disintegration.

In process control specifications for the Rasagiline Base DR 1mg tablet cores are:

| Parameter | Minimum | Target | Maximum |
|---|---|---|---|
| Avarage weight (mg) | 111 | 117 (121 Actual) | 123 |
| Individual weight (mg) | 111 | 117 | 123 |
| Tickness (mm) | 3.3 | 3.6 (3.7 Actual) | 3.9 |
| Hardness (SCU) | 7 | 9 (10 Actual) | 11 |
| Friability (%) | -- | -- | 1.0 |
| Disintegration (minutes) | -- | -- | 5 |

The tablet cores are weighed and the percentage yield is calculated.

### VII. Sub-coating:

Tablet cores were first coated with hypromellose (Pharmacoat 606) as a pre-coating, followed by Methacrylic Acid - Methyl Methacrylate Copolymer [1:1] (Eudragit® L30D-55, 30% dispersion of Eudragit® L100-55) to prevent any possible interaction between the Rasagiline base in the core and the Eudragit L polymer.
1. Preparation of Pharmacoat 606 solution:
   Pharmacoat 606 (hypromellose USP) solution was prepared using Pharmacoat 606 in purified water in a weight ratio of 1:10.
2. Pre heating:
   The tablet cores are place in an Ohara Coater coating pan the tablets was heated under inlet air temperature of 50°C (45° to 55°C) and outlet air temperature of 40° to 50°C.
3. Spraying process:
   The tablet cores were sprayed with solution in an Ohara Coater coating pan. The inlet air temperature was 50°C (in the range of 40-50°C); the outlet air temperature was in range of 30-40°C. The pan speed was set to 16 rpm in the range of 14-18rpm; spraying rate was 15-35 gr/min. The tablets were dried for 1 hour with inlet air temperature of 45°C (in the range of 40-50°C).

### VIII.Enteric Coating:

The Rasagiline subcoated drug product tablet formulation described in previous section was used for the enteric coated.
1. Preparation of Eudragit® L100-55 dispersion:
   Triethyl citrate was mixed with the water for 15 min.
      The Talc Extra fine was added into the Triethyl citrate and water dispersion in an Ultraturax within 10 minutes.
   Eudragit® L100-55 was added to Triethyl citrate/talc dispersion, filtered and stirred to the continuation of the process.
2. Pre heating:
   The tablet cores are place in an Ohara Coater coating pan the tablets was heated under inlet air temperature of 50°C (45° to 55°C) and outlet air temperature of 45°C (40° to 50°C).
2. Spraying process:
   The tablets were sprayed with the dispersion in an Ohara coater pan. The inlet air temperature was 45°C; the outlet air temperature was 35°C (in range of 30-40°C). The pan speed was set to 16 rpm (in the range of 14-18 rpm), and the spraying rate was 5-20 gr/min.
      The tablets were dried for 2 hours; with inlet air temperature of 50°C (in the range of 45-55°C), on minimum pan speed.

### Example 6b - 1.0 mg rasagiline base, 76 mg core tablet weight

This formulation was prepared using similar steps as described in example 6a.

**Table 6b: composition of rasagiline-base delayed release enteric coated tablet**

| **Component** | **Function** | **Per Tablet** **(mg)** |
|---|---|---|
| Core tablets | | |
| Rasagiline base | Drug Substance | 1.0 |
| Malic acid | Antioxidant/ Stabilizer | 1.6 |
| Mannitol | Filler | 45.0 |
| Aerosil | Flowing Agent | 0.4 |
| Starch NF | Binder | 5.0 |
| Starch, Pregelatinized (Starch STA-RX 1500) | Disintegrant | 20.0 |
| Talc | Lubricant | 1.5 |
| Stearic Acid | Lubricant | 1.5 |
| **Total Core Tablet Weight** | | **76.0** |
| Subcoating | | |
| Pharmacoat® 606 (Hypromellose USP) Granules | Coating Agent | 3.5 |
| Purified Water | Processing Agent | |
| Coating Suspension | | |
| Eudragit® L-30D-55 | Coating Agent | 4.0* |
| Talc USP Extra Fine | Lubricant | 1.9 |
| Triethyl citrate NF | Plasticizer | 0.8 |
| Purified Water | Processing Agent | |
| Total Tablet weight | | **86.2** |

| | | |
|---|---|---|
| * Dry substance remaining on the core. | | |

### Example 6c - 0.5 mg rasagiline base, 117 mg core tablet weight

This formulation was prepared using similar steps as described in example 6a.

**Table 6c: composition of rasagiline-base delayed release enteric coated tablet**

| **Component** | **Function** | **Per Tablet** **(mg)** |
|---|---|---|
| Core tablets | | |
| Rasagiline base | Drug Substance | 0.5 |
| Malic acid | Antioxidant/ Stabilizer | 1.6 |
| Mannitol | Filler | 80.34 |
| Aerosil | Flowing Agent | 0.6 |
| Starch NF | Binder | 10.0 |
| Starch, Pregelatinized (Starch STA-RX 1500) | Disintegrant | 20.0 |
| Talc | Lubricant | 2.0 |
| Stearic Acid | Lubricant | 2.0 |
| **Total Core Tablet Weight** | | **117.0** |
| Subcoating | | |
| Pharmacoat® 606 (Hypromellose USP) Granules | Coating Agent | 4.8 |
| Purified Water | Processing Agent | |
| Coating Suspension | | |
| Eudragit® L-30D-55 | Coating Agent | 6.25* |
| Talc USP Extra Fine | Lubricant | 3.1 |
| Triethyl citrate NF | Plasticizer | 1.25 |
| Purified Water | Processing Agent | |
| **Total Tablet Weight** | | **132.4** |

| | | |
|---|---|---|
| * Dry substance remaining on the core. | | |

### Example 6d. Preparation of 0.5 mg rasagiline base delayed release enteric coated tablet

In this example, a 0.5 mg rasagiline base delayed release enteric coated tablet containing malic acid (76 mg core tablet weight) was prepared using similar steps as described in example 6a.

**Table 6d: composition of rasagiline-base delayed release enteric coated tablet**

| **Component** | **Function** | **Per Tablet** **(mg)** |
|---|---|---|
| Core tablets | | |
| Rasagiline base | Drug Substance | 0.5 |
| Malic acid | Antioxidant/ Stabilizer | 1.6 |
| Mannitol | Filler | 45.5 |
| Aerosil | Flowing Agent | 0.4 |
| Starch NF | Binder | 5.0 |
| Starch, Pregelatinized (Starch STA-RX 1500) | Disintegrant | 20.0 |
| Talc | Lubricant | 1.5 |
| Stearic Acid | Lubricant | 1.5 |
| **Total Core Tablet Weight** | | **76.0** |
| Subcoating | | |
| Pharmacoat® 606 (Hypromellose USP) Granules | Coating Agent | 3.5 |
| Purified Water | Processing Agent | |
| Coating Suspension | | |
| Eudragit® L-30D-55 | Coating Agent | 4.0* |
| Talc USP Extra Fine | Lubricant | 1.9 |
| Triethyl citrate NF | Plasticizer | 0.8 |
| Purified Water | Processing Agent | |
| **Total Tablet weight** | | **86.2** |

| | | |
|---|---|---|
| * Dry substance remaining on the core. | | |

### Example 7. Stability Results of Tablets Prepared According to Example 6a

Stability of enteric coated tablets produced using formulations containing citric acid was tested under different storage conditions. The results are summarized below.

### Stability results (Accelerated Conditions):

The dissolution profile of the enteric coated tablets in 0.1N HCl was acceptable according to USP specification for delayed release (enteric coated) articles, 29th edition, Chapter 724, showing less than 10% release after 120 minutes.

The following tables show that analytical results for tablets under various storage conditions.

**Tablet Cores:**

| Conditions | | Assay (%) | Total Impurities |
|---|---|---|---|
| T=0 | | 101.2 | <DL |
| 40°C, 75RH | 1Mo | 101.1 | 0.1 |
| | 2Mo | 98.3 | 0.3 |
| | 3Mo | 93.3 | 0.5 |
| | 4Mo | 93.1 | 0.4 |
| 30°C, 65RH | 1Mo | 101.4 | <DL |
| | 2Mo | 101.9 | <QL |
| | 3Mo | 98.3 | <QL |
| 25°C, 60RH | 1Mo | 101.5 | <DL |
| | 2Mo | 102.0 | <QL |
| | 3Mo | 100.3 | <QL |

**Enteric Coated Tablets:**

| Conditions | | Assay % | Total Impurities |
|---|---|---|---|
| T=0 | | 98.2 | <QL |
| 40°C, 75RH | 1Mo | 100.5 | 0.2 |
| | 2Mo | 96.4 | 0.3 |
| | 3Mo | 96.6 | 0.5 |
| 30°C, 65RH | 1Mo | 98.2 | <QL |
| | 2Mo | 100.2 | <QL |
| | 3Mo | 101.0 | 0.1 |
| 25°C, 60RH | 1Mo | 101.5 | <QL |
| | 2Mo | 96.7 | <QL |
| | 3Mo | 99.5 | <QL |

**N-(2-Chloroallyl)-1(R)-aminoindan (2-Cl-AAI) Impurities**

| Batch No | 2-Cl-AAI Content, % |
|---|---|
| 1 | < 0.00004 |

### Example 8. Preparation of Rasagiline Base Tablet Cores with Citric Acid

| Raw material | mg/ tablet | Percentage |
|---|---|---|
| Part I, Granulation solution | | |
| Citric acid | 1.6 | 2.0 |
| Rasagiline base | 1 | 1.25 |
| Purified Water | 12.35 | 15.44 |
| Part II | | |
| Mannitol | 48.5 | 60.63 |
| Aerosil 200 | 0.18 | 0.22 |
| Starch NF/BP | 6.1 | 7.62 |
| Pregelatinized starch NF/Ph.Eur | 20.0 | 25.0 |
| Part III | | |
| Aerosil 200 | 0.18 | 0.22 |
| Part IV | | |
| Stearic acid | 1.22 | 1.52 |
| Talc | 1.22 | 1.52 |
| **Total:** | **80.0** | **100** |

The above composition can also be used to prepare rasagiline base tablets with malic acid by replacing the citric acid with the same amount of malic acid.

Calculated amounts of external excipients in accordance with actual amount of granulate:

| mg/tablet | Raw material | Percentage |
|---|---|---|
| | Part III | |
| | Granulate | |
| 0.18 | Aerosil 200 | 0.22 |
| | Part IV | |
| 1.22 | Stearic acid | 1.52 |
| 1.22 | Talc | 1.52 |

### I. Granulation solution preparation:

1. Weigh 80% of needed amount of Purified water into glass.
2. Weigh into the same glass Citric acid.
3. Insert stirrer into the glass and start to stir up to complete solubility about 5-10 minutes.
4. Weigh.Rasagiline base and add it into the obtained Citric acid solution.
5. Continue stirring about 30 minutes to complete solubility of API.

### II. Granulation preparation:

1. Weigh Mannitol, Aerosil 200, Starch and Pregelatinized starch and transfer all excipients to Diosna P-6 (Diosna) and mix for 1 minute with Mixer I (270 rpm).
2. Mix the excipient for 1 addition minute with Mixer I (270 rpm) and Chopper I (1500 rpm)
3. Add Granulation solution into the Diosna P-6(Diosna) and mix for 2 minutes with Mixer II (540rpm) and Chopper II (2200 rpm).
4. Clean glass after granulation solution with 46.563 g of Purified water and add it into the Diosna P-6 (Diosna).
5. Mix for 2 minutes with Mixer II (540rpm) and Chopper II (2200 rpm).
6. Transfer obtained granulate into the Glatt 1.1 (Fluid Bed) for drying at 37°C inlet air up to L.O.D. NMT 1.5%.

### Conditions of drying:

Inlet: Min. - 35°C; Target - 50°C; Max. - 55°C
Outet: Product temperature - 37°C
Flow: Min. - 25; Target - 60; Max. - 1000

### III. Milling:

Mill granulate through 0.6 mm sieve using Frewitt.

### IV. Final blend:

1. Weigh obtained amount of granulate.
2. Calculate amounts of Aerosil 200, Stearic acid and Talc in accordance with actual granulation weight.
3. Screen Aerosil 200 through 50 mesh sieve.
4. Weigh needed amount of Aerosil 200 after sieving.
5. Transfer milled granulate and Aerosil 200 after sieving into the Y-cone.
6. Mix for 2 minutes.
7. Weigh Stearic acid and Talc.
8. Screen these excipients through 50 mesh sieve.
9. Transfer them into the Y-cone.
10. Mix for 5 minutes.

### V. Tablet compression:

Machine: Sviac
Diameter of punch: 5.0 mm (it may be changed ±10%)
Tablet weight - 80 mg± 5%
Hardness: 3-7 kP
Friability: Not More Than 1%
Disintegration: Not More Than 5 minutes

### Example 9. Preparation of Rasagiline Base Tablet Cores with Malic Acid

| Raw material | mg/ tablet | Percentage |
|---|---|---|
| Part I, Granulation solution | | |
| Malic acid | 1.6 | 3.72 |
| Rasagiline base | 1 | 2.33 |
| Part II | | |
| Mannitol | 25.8 | 60.0 |
| Aerosil 200 | 0.1 | 0.24 |
| Starch NF/BP | 3.0 | 6.98 |
| Pregelatinized starch NF/Ph.Eur | 10.0 | 23.26 |
| Part III | | |
| Aerosil 200 | 0.1 | 0.23 |
| Part IV | | |
| Stearic acid | 0.7 | 1.63 |
| Talc | 0.7 | 1.63 |
| **Total:** | **43.0** | **100** |

The above composition can also be used to prepare rasagiline base tablets with citric acid by replacing the malic acid with the same amount of citric acid.

### I. Granulation solution preparation.

1. Weigh 80% of needed amount of Purified water into glass.
2. Weigh Malic acid and add it into the same glass.
3. Insert stirrer into the glass and start to stir up to complete solubility about 5-10 minutes.
4. Weigh Rasagiline base and add it into the obtained Malic acid solution.
5. Continue stirring about 30 minutes to complete solubility of API.

### II. Granulation preparation.

1. Weigh Mannitol, Aerosil 200, Starch and Pregelatinized starch and transfer all excipients to Diosna P-10 (Diosna) and mix for 1 minute with Mixer I.
2. Mix the excipient for 1 addition minute with Mixer I and Chopper I rpm.
3. Add granulation solution into the Diosna P-10 (Diosna) and mix for 2 minutes with Mixer II and Chopper II.
4. Add additional Purified Water into the Diosna P-10 (Diosna) and mix for 2 minutes with Mixer II, and Chopper II.
5. Transfer obtained granulate into the Glatt 5 (Fluid Bed)for drying at 37°C inlet air up to L.O.D. NMT 1.5%.
   Conditions of drying:
   Inlet: Min. - 35°C; Target - 50°C; Max. - 55°C
   Outet: Product temperature - 37°C

### III. Milling.

Weigh and add Aerosil 200 to granulate and milled granulate through 0.6 mm sieve using Frewitt.

### IV. Final blend.

1. Weigh Stearic acid and Talc.
2. Screen the excipients through 50 mesh sieve.
3. Transfer milled granulate and sieved Stearic acid and Talc into the Y-cone.
4. Mix for 5 minutes.

### V. Tablet compression:

Machine: Sviac
Diameter of punch: 4.0 mm (it may be changed ±10%)
Tablet weight - 43 mg± 5%
Hardness: 3-5 kP
Friability: Not More Than 1%
Disintegration: Not More Than 5 minutes

### Example 10. Preparation of Rasagiline Base Tablet Cores with Both Citric and Malic acids

| Raw material | Mg/ tablet | Percentage |
|---|---|---|
| Part I,Granulation solution | | |
| Citric acid | 0.8 | 0.68 |
| Malic acid | 0.8 | 0.68 |
| Rasagiline base | 1.0 | 0.85 |
| Part II | | |
| Mannitol | 79.8 | 68.2 |
| Aerosil 200 | 0.3 | 0.26 |
| Starch NF/BP | 10.0 | 8.55 |
| Pregelatinized starch NF/Ph.Eur | 20.0 | 17.09 |
| Part III | | |
| Aerosil 200 | 0.3 | 0.26 |
| Part IV | | |
| Stearic acid | 2.0 | 1.71 |
| Talc | 2.0 | 1.71 |
| **Total:** | **117.0** | **100** |

### Calculated amounts of external excipients in accordance with actual amount of granulate

| Raw material | mg/ tablet | Percentage |
|---|---|---|
| Part III | | |
| Granulate | | |
| Aerosil 200 | 0.3 | 0.26 |
| Part IV | | |
| Stearic acid | 2.0 | 1.71 |
| Talc | 2.0 | 1.71 |

### I. Granulation solution 1 preparation.

1. Weigh 80% of needed amount of Purified water into glass.
2. Weigh into the same glass Citric acid.
3. Insert stirrer into the glass and start to stir up to complete solubility about 5-10 minutes.
4. Weigh Rasagiline base and add it into the obtained Citric acid solution.
5. Continue stirring about 30 minutes to complete solubility of API.

### II. Granulation solution 2 preparation.

1. Weigh 20% of needed amount of Purified water into the glass.
2. Add into this glass weighed amount of Malic acid.
3. Insert stirrer into the glass and start to stir up to complete solubility about 5-10 minutes.

### III. Granulation preparation.

1. Weigh Mannitol, Aerosil 200, Starch and Pregelatinized starch and transfer all excipients to Diosna P-6 (Diosna) and mix for 1 minute with Mixer I (270 rpm).
2. Mix the excipient for 1 addition minute with Mixer I (270 rpm) and Chopper I (1500 rpm)
3. Add Granulation solution 1 into the Diosna P-6 (Diosna) and mix for 2 minutes with Mixer II (540rpm) and Chopper II (2200 rpm).
4. Clean glass after granulation solution 1 with Granulation solution 2 and add it into the Diosna P-6(Diosna).
5. Mix for 2 minutes with Mixer II (540rpm) and Chopper II (2200 rpm).
6. Transfer obtained granulate into the Glatt 1.1 (Fluid Bed) for drying at 37°C inlet air up to L.O.D. NMT 1.5%.
   Conditions of drying:
   Inlet: Min. - 35°C; Target - 50°C; Max - 55°C
   Outet: Product temperature - 37°C
   Flow: Min. - 25; Target - 60; Max - 1000

### IV. Milling:

Mill obtained granulate through 0.6 mm sieve using Frewitt.

### V. Final blend:

1. Weigh obtained amount of granulate.
2. Calculate amounts of Aerosil 200, Stearic acid and Talc in accordance with actual granulation weight.
3. Screen Aerosil 200 through 50 mesh sieve.
4. Weigh needed amount of Aerosil 200 after sieving.
5. Transfer milled granulate and Aerosil 200 after sieving into the Y-cone.
6. Mix for 2 minutes.
7. Weigh Stearic acid and Talc.
8. Screen these excipients through 50 mesh sieve.
9. Transfer them into the Y-cone.
10. Mix for 5 minutes.

### VI. Tablet compression

Machine: Sviac
Diameter of punch: 6.0 mm (it may be changed ±10%)
Tablet weight - 117 mg± 5%
Hardness: 6-8 kP
Friability: Not More Than 1%
Disintegration: Not More Than 5 minutes

### VII. Subcoating:

| Mg/tablet | Raw material |
|---|---|
| 4.8 | Pharmacoat 606 (Hypromellose USP) |

Equipment: O'HARA, Peristaltic pump
1. Preparation of Sub-coating solution:
   Pharmacoat 606 (hypromellose USP) was added into the vessel with 1510g of Purified water and mixed for 30 minutes using stirrer.
2. Preheating:
   The core tablets were placed into the Pan 2.5 kg of O'HARA Coater and preheated:
      Inlet air temperature - 50°C (45° to 55°C)
      Outlet air temperature - 45°C (40° to 50°C).
      Difference pressure - -50Pa
3. Spraying process (the process was continued till desired tablet weight was achieved):
   Sub-coating solution was sprayed on the preheated core tablets at the following conditions:
      Number of spray guns - 1
      Nozzle bore -1 mm
      Distance tablet bed/spray gun - 15 cm
      Pan speed 10 rpm (8-12 rpm)
         Inlet air temperature - 50°C (45° to 55°C)
      Outlet air temperature - 35°C (30° to 40°C)
      Spraying rate - 10-20 g/min
      Difference pressure - -50Pa
      Atomizing air pressure - 30 Psi
      Pattern air pressure - 30 Psi
4. Drying process:
   Inlet air temperature - 45°C (40° to 50°C)
   Outlet air temperature - 40°C-50°C
   Pan speed - 5 rpm Jogging
   Drying time - 60 min

### Example 11. Additional Rasagiline Base Enteric Coated Formulations with Citric Acid

### Example 11a - 0.5 mg Rasagiline base

This example describes 0.5 mg rasagiline base formulations with variations in the amount of citric acid and other excipients. These formulations have a dissolution and pharmacokinetic profile (Cₘₐₓ and AUC) resembling that of example 1.

| **Component** | **Function** | **Per Tablet** **(mg)** | **Per Tablet** **(mg)** | **Per Tablet** **(mg)** | **Per Tablet** **(mg)** |
|---|---|---|---|---|---|
| Core tablets | | | | | |
| Rasagiline base | Drug Substance | 0.5 | 0.5 | 0.5 | 0.5 |
| Citric acid | Antioxidant | 1.6 or 0.8 | 1.6 or 0.8 | 1.6 or 0.8 | 1.6 or 0.8 |
| Mannitol | Filler | 45.5 | 68.3 | 50.5 | 80.3 |
| Aerosil | Flowing Agent | 0.4 | 0.6 | 0.4 | 0.6 |
| Starch NF | Binder | 5.0 | 10.0 | 5.0 | 10 |
| Starch, Pregelatinized (Starch STA-RX 1500) | Disintegrant | 20.0 | 20.0 | 15.0 | 20.0 |
| Talc | Lubricant | 1.5 | 2.0 | 1.5 | 2.0 |
| Stearic Acid | Lubricant | 1.5 | 2.0 | 1.5 | 2.0 |
| **Total Core Tablet Weight** | | **76.0**(+/-10%) | **105.0(**+/-10%) | **76.0**(+/-10%) | **117.0** (+/-10%) |
| Subcoating | | | | | |
| Pharmacoat 606 (Hypromellose USP) Granules | Coating Agent | 3.5(+/-10%) | 4.8(+/-10%) | 3.5(+/-10%) | 4.8(+/-10%) |
| Purified Water | Processing Agent | | | | |
| Coating Suspension | | | | | |
| Eudragit L-30D-55 | Coating Agent | 4.0(+/-10%) | 4.0(+/-10%) | 4.0(+/-10%) | 6.25(+/-10%) |
| Talc USP Extra Fine | Lubricant | 1.9(+/-10%) | 1.9(+/-10%) | 1.9(+/-10%) | 3.1(+/-10%) |
| Triethyl citrate NF | Plasticizer | 0.8(+/-10%) | 0.8(+/-10%) | 0.8(+/-10%) | 1.25(+/-10%) |
| Purified Water | Processing Agent | | | | |

### Example 11b - 1.0 mg Rasagiline base

This example describes 1 mg rasagiline base formulations with variations in the amount of citric acid and other excipients. These formulations have a dissolution and pharmacokinetic profile (Cₘₐₓ and AUC) resembling that of example 1.

| **Component** | **Function** | **Per Tablet** **(mg)** | **Per Tablet (mg** | **Per Tablet** **(mg)** | **Per Tablet (mg** |
|---|---|---|---|---|---|
| Core tablets | | | | | |
| Rasagiline base | Drug Substance | 1.0 | 1.0 | 1.0 | 1.0 |
| Citric acid | Antioxidant | 1.6 or 0.8 | 1.6 or 0.8 | 1.6 or 0.8 | 1.6 or 0.8 |
| Mannitol | Filler | 45.0 | 67.8 | 50.0 | 79.8 |
| Aerosil | Flowing Agent | 0.4 | 0.6 | 0.4 | 0.6 |
| Starch NF | Binder | 5.0 | 10.0 | 5.0 | 10.0 |
| Starch, Pregelatinized (Starch STA-RX 1500) | Disintegrant | 20.0 | 20.0 | 15.0 | 20.0 |
| Talc | Lubricant | 1.5 | 2.0 | 1.5 | 2.0 |
| Stearic Acid | Lubricant | 1.5 | 2.0 | 1.5 | 2.0 |
| **Total Core** **Tablet Weight** | | **76.0**(+/-10%) | **105.0**(+/-10%) | **76.0**(+/-10%) | **117.0**(+/-10%) |
| Subcoating | | | | | |
| Pharmacoat 606 (Hypromellose USP) Granules | Coating Agent | 3.5(+/-10%) | 4.8(+/-10%) | 3.5(+/-10%) | 4.8(+/-10%) |
| Purified Water | Processing Agent | | | | |
| Coating Suspension | | | | | |
| Eudragit L-30D-55 | Coating Agent | 4.0(+/-10%) | 4.0(+/-10%) | 4.0(+/-10%) | 6.25(+/-10%) |
| Talc USP Extra Fine | Lubricant | 1.9(+/-10%) | 1.9(+/-10%) | 1.9(+/-10%) | 3.1(+/-10%) |
| Triethyl citrate NF | Plasticizer | 0.8(+/-10%) | 0.8(+/-10%) | 0.8(+/-10%) | 1.25(+/-10%) |
| Purified Water | Processing Agent | | | | |

### Example 12. Additional Rasagiline Base Enteric Coated Formulations with Malic Acid

### Example 12a - 0.5 mg Rasagiline base

This example describes 0.5 mg rasagiline base formulations with variations in the amount of malic acid and other excipients. These formulations have a dissolution and pharmacokinetic profile (Cₘₐₓ and AUC) resembling that of example 1.

| Component | **Function** | **Per Tablet** **(mg)** | **Per Tablet** **(mg)** | **Per Tablet (m** | **Per Tablet (mg** |
|---|---|---|---|---|---|
| Core tablets | | | | | |
| Rasagiline base | Drug Substance | 0.5 | 0.5 | 0.5 | 0.5 |
| Malic acid | Antioxidant | 1.6 or 0.8 | 1.6 or 0.8 | 1.6 or 0.8 | 1.6 or 0.8 |
| Mannitol | Filler | 45.5 | 68.3 | 50.5 | 80.3 |
| Aerosil | Flowing Agent | 0.4 | 0.6 | 0.4 | 0.6 |
| Starch NF | Binder | 5.0 | 10.0 | 5.0 | 10 |
| Starch, Pregelatinized (Starch STA-RX 1500) | Disintegrant | 20.0 | 20.0 | 15.0 | 20.0 |
| Talc | Lubricant | 1.5 | 2.0 | 1.5 | 2.0 |
| Stearic Acid | Lubricant | 1.5 | 2.0 | 1.5 | 2.0 |
| **Total Core Tablet Weight** | | **76.0**(+/-10%) | **105.0**(+/-10%) | **76.0**(+/-10%) | **117.0**(+/-10%) |
| Subcoating | | | | | |
| Pharmacoat 606 (Hypromellose USP) Granules | Coating Agent | 3.5(+/-10%) | 4.8(+/-10%) | 3.5(+/-10%) | 4.8(+/-10%) |
| Purified Water | Processing Agent | | | | |
| Coating Suspension | | | | | |
| Eudragit L-30D-55 | Coating Agent | 4.0(+/-10%) | 4.0(+/-10%) | 4.0(+/-10%) | 6.25(+/-10%) |
| Talc USP Extra Fine | Lubricant | 1.9(+/-10%) | 1.9(+/-10%) | 1.9(+/-10%) | 3.1(+/-10%) |
| Triethyl citrate NF | Plasticizer | 0.8(+/-10%) | 0.8(+/-10%) | 0.8(+/-10%) | 1.25(+/-10%) |
| Purified Water | Processing Agent | | | | |

### Example 12b - 1.0 mg Rasagiline base

This example describes 1 mg rasagiline base formulations with variations in the amount of malic acid and other excipients. These formulations have a dissolution and pharmacokinetic profile (Cₘₐₓ and AUC) resembling that of example 1.

| **Component** | **Function** | **Per Tablet** **(mg)** | **Per Tablet** **(mg)** | **Per Tablet** **(mg)** | **Per Tablet (mg** |
|---|---|---|---|---|---|
| Core tablets | | | | | |
| Rasagiline base | Drug Substance | 1.0 | 1.0 | 1.0 | 1.0 |
| Malic acid | Antioxidant | 1.6 or 0.8 | 1.6 or 0.8 | 1.6 or 0.8 | 1.6 or 0.8 |
| Mannitol | Filler | 45.0 | 67.8 | 50.0 | 79.8 |
| Aerosil | Flowing Agent | 0.4 | 0.6 | 0.4 | 0.6 |
| Starch NF | Binder | 5.0 | 10.0 | 5.0 | 10.0 |
| Starch, Pregelatinized (Starch STA-RX 1500) | Disintegrant | 20.0 | 20.0 | 15.0 | 20.0 |
| Talc | Lubricant | 1.5 | 2.0 | 1.5 | 2.0 |
| Stearic Acid | Lubricant | 1.5 | 2.0 | 1.5 | 2.0 |
| **Total Core Tablet Weight** | | **76.0**(+/-10%) | **105.0**(+/-10%) | **76.0**(+/-10%) | **117.0**(+/-10%) |
| Subcoating | | | | | |
| Pharmacoat 606 (Hypromellose USP) Granules | Coating Agent | 3.5(+/-10%) | 4.8(+/-10%) | 3.5(+/-10%) | 4.8(+/-10%) |
| Purified Water | Processing Agent | | | | |
| Coating Suspension | | | | | |
| Eudragit L-30D-55 | Coating Agent | 4.0(+/-10%) | 4.0(+/-10%) | 4.0(+/-10%) | 6.25(+/-10%) |
| Talc USP Extra Fine | Lubricant | 1.9(+/-10%) | 1.9(+/-10%) | 1.9(+/-10%) | 3.1(+/-10%) |
| Triethyl citrate NF | Plasticizer | 0.8(+/-10%) | 0.8(+/-10%) | 0.8(+/-10%) | 1.25(+/-10%) |
| Purified Water | Processing Agent | | | | |

### Example 13. Additional Rasagiline Base Enteric Coated Formulations with both Citric and Malic Acid

### Example 13a - 0.5 mg Rasagiline base

This example describes 0.5 mg rasagiline base formulations with variations in the amount of citric acid, malic acid, and other excipients. These formulations have a dissolution and pharmacokinetic profile (Cₘₐₓ and AUC) resembling that of Example 1.

| **Component** | **Function** | **Per Tablet** **(mg)** | **Per Tablet** **(mg)** | **Per Tablet** **(mg)** | **Per Tablet** **(mg)** |
|---|---|---|---|---|---|
| Core tablets | | | | | |
| Rasagiline base | Drug Substance | 0.5 | 0.5 | 0.5 | 0.5 |
| Malic acid | Antioxidant | 0.8 or 0.4 | 0.8 or 0.4 | 0.8 or 0.4 | 1.6 or 0.8 |
| Citric acid | Antioxidant | 0.8 or 0.4 | 0.8 or 0.4 | 0.8 or 0.4 | 1.6 or 0.8 |
| Mannitol | Filler | 45.5 | 68.3 | 50.5 | 80.3 |
| Aerosil | Flowing Agent | 0.4 | 0.6 | 0.4 | 0.6 |
| Starch NF | Binder | 5.0 | 10.0 | 5.0 | 10.0 |
| Starch, Pregelatinized (Starch STA-RX 1500) | Disintegrant | 20.0 | 20.0 | 15.0 | 20.0 |
| Talc | Lubricant | 1.5 | 2.0 | 1.5 | 2.0 |
| Stearic Acid | Lubricant | 1.5 | 2.0 | 1.5 | **2.0** |
| **Total Core Tablet Weight** | | **76.0**(+/-10%) | **105.0**(+/-10%) | **76.0**(+/-10%) | **117.0**(+/-10%) |
| Subcoating | | | | | |
| Pharmacoat 606 (Hypromellose USP) Granules | Coating Agent | 3.5(+/-10%) | 4.8(+/-10%) | 3.5(+/-10%) | 4.8(+/-10%) |
| Purified Water | Processing Agent | | | | |
| Coating Suspension | | | | | |
| Eudragit L-30D-55 | Coating Agent | 4.0(+/-10%) | 4.0(+/-10%) | 4.0(+/-10%) | 6.25(+/-10%) |
| Talc USP Extra Fine | Lubricant | 1.9(+/-10%) | 1.9(+/-10%) | 1.9(+/-10%) | 3.1(+/-10%) |
| Triethyl citrate NF | Plasticizer | 0.8(+/-10%) | 0.8(+/-10%) | 0.8(+/-10%) | 1.25(+/-10%) |
| Purified Water | Processing Agent | | | | |

### Example 13b - 1.0 mg Rasagiline base

This example describes 1 mg rasagiline base formulations with variations in the amount of citric acid, malic acid, and other excipients. These formulations have a dissolution and pharmacokinetic profile (Cₘₐₓ and AUC) resembling that of example 1.

| **Component** | **Function** | **Per Tablet** **(mg)** | **Per Tablet** **(mg)** | **Per Tablet** **(mg)** | **Per Tablet** **(mg)** |
|---|---|---|---|---|---|
| Core tablets | | | | | |
| Rasagiline base | Drug Substance | 1.0 | 1.0 | 1.0 | 1.0 |
| Malic acid | Antioxidant | 0.8 or 0.4 | 0.8 or 0.4 | 0.8 or 0.4 | 1.6 or 0.8 |
| Citric acid | Antioxidant | 0.8 or 0.4 | 0.8 or 0.4 | 0.8 or 0.4 | 1.6 or 0.8 |
| Mannitol | Filler | 45.0 | 67.8 | 50.0 | 79.8 |
| Aerosil | Flowing Agent | 0.4 | 0.6 | 0.4 | 0.6 |
| Starch NF | Binder | 5.0 | 10.0 | 5.0 | 10.0 |
| Starch, Pregelatinized (Starch STA-RX 1500) | Disintegrant | 20.0 | 20.0 | 15.0 | 20.0 |
| Talc | Lubricant | 1.5 | 2.0 | 1.5 | 2.0 |
| Stearic Acid | Lubricant | 1.5 | 2.0 | 1.5 | **2.0** |
| **Total Core Tablet Weight** | | **76.0**(+/-10%) | **105.0**(+/-10%) | **76.0**(+/-10%) | **117.0**(+/-10%) |
| Subcoating | | | | | |
| Pharmacoat 606 (Hypromellose USP) Granules | Coating Agent | 3.5(+/-10%) | 4.8(+/-10%) | 3.5(+/-10%) | 4.8(+/-10%) |
| Purified Water | Processing Agent | | | | |
| Coating Suspension | | | | | |
| Eudragit L-30D-55 | Coating Agent | 4.0(+/-10%) | 4.0(+/-10%) | 4.0(+/-10%) | 6.25(+/-10%) |
| Talc USP Extra Fine | Lubricant | 1.9(+/-10%) | 1.9(+/-10%) | 1.9(+/-10%) | 3.1(+/-10%) |
| Triethyl citrate NF | Plasticizer | 0.8(+/-10%) | 0.8(+/-10%) | 0.8(+/-10%) | 1.25(+/-10%) |
| Purified Water | Processing Agent | | | | |

### Example 14. Color Coated Rasagiline Base Enteric Coated Formulation with Citric Acid

### Example 14a - 0.5 mg Rasagiline base

This example describes a 0.5 mg rasagiline base formulation containing citric acid with an extra color coating.

| **Component** | **Function** | **Per Tablet** **(mg)** |
|---|---|---|
| Core tablets | | |
| Rasagiline base | Drug Substance | 0.5 |
| Citric acid | Antioxidant | 1.6 |
| Mannitol | Filler | 80.3 |
| Aerosil | Flowing Agent | 0.6 |
| Starch, Pregelatinized (Starch STA-RX 1500) | Disintegrant | 20.0 |
| Starch NF | Binder | 10.0 |
| Talc | Lubricant | 2.0 |
| Stearic Acid | Lubricant | 2.0 |
| **Total Core Tablet Weight** | | **117.0** |
| Subcoating | | |
| Pharmacoat 606 (Hypromellose USP) Granules | Coating Agent | 4.8 |
| Purified Water | Processing Agent | |
| Coating Suspension | | |
| Eudragit L-30D-55 | Coating Agent | 6.25* |
| Talc USP Extra Fine | Lubricant | 3.1 |
| Triethyl citrate NF | Plasticizer | 1.25 |
| Purified Water | Processing Agent | |
| Top coat | | |
| **OPADRY II OY-GM-28900 WHITE** **(catnum. 415850005)** **OR** **OPADRY II Y-30-18037 WHITE** **(catnum. 415880719)** **OR/AND** **Opadry fx 63f97546 silver** | Coating Agent | **1-5** |
| Purified Water | Processing Agent | |

| | | |
|---|---|---|
| * Dry substance remaining on the core. | | |

### Example 14b - 1.0 mg Rasagiline base

This example describes a 1 mg rasagiline base formulation containing citric acid with an extra color coating.

| **Component** | **Function** | **Per Tablet** **(mg)** |
|---|---|---|
| Core tablets | | |
| Rasagiline base | Drug Substance | 1.0 |
| Citric acid | Antioxidant | 1.6 |
| Mannitol | Filler | 79.8 |
| Aerosil | Flowing Agent | 0.6 |
| Starch, Pregelatinized (Starch STA-RX 1500) | Disintegrant | 20.0 |
| Starch NF | Binder | 10.0 |
| Talc | Lubricant | 2.0 |
| Stearic Acid | Lubricant | 2.0 |
| **Total Core Tablet Weight** | | **117.0** |
| Subcoating | | |
| Pharmacoat 606 (Hypromellose USP) Granules | Coating Agent | 4.8 |
| Purified Water | Processing Agent | |
| Coating Suspension | | |
| Eudragit L-30D-55 | Coating Agent | 6.25* |
| Talc USP Extra Fine | Lubricant | 3.1 |
| Triethyl citrate NF | Plasticizer | 1.25 |
| Purified Water | Processing Agent | |
| Top coat | | |
| **Opadry® II 31F20721 Blue** **OR** **Opadry® II 34G24627 Pink** **OR/AND** **Opadry fx 63f97546 silver** | Coating Agent | **1-5** |
| Purified Water | Processing Agent | |

| | | |
|---|---|---|
| * Dry substance remaining on the core. | | |

### Example 14c - Rasagiline base (Formulation III with color coating)

This example describes rasagiline base formulation (Formulation III) containing citric acid with an extra color coating.

| **Component** | **Function** | **Per Tablet** **(mg)** - 0.5 mg rasagiline formulation | **Per Tablet** **(mg)** - 1.0 mg rasagiline formulation |
|---|---|---|---|
| Core tablets | | | |
| Rasagiline base | Drug Substance | 0.5 | 1 |
| Citric acid | Antioxidant | 1.6 | 1.6 |
| Mannitol | Filler | 45.5 | 45 |
| Aerosil | Flowing Agent | 0.4 | 0.4 |
| Starch NF | Binder | 5 | 5 |
| Starch, Pregelatinized (Starch STA-RX 1500) | Disintegrant | 20 | 20 |
| Talc | Lubricant | 1.5 | 1.5 |
| Stearic Acid | Lubricant | 1.5 | 1.5 |
| **Total Core Tablet Weight** | | **76** | **76** |
| Subcoating | | | |
| Pharmacoat 606 (Hypromellose USP) Granules | Coating Agent | 3.5 | 3.5 |
| Coating Suspension | | | |
| Eudragit L-30D-55 | Coating Agent | 4 | 4 |
| Talc USP Extra Fine | Lubricant | 1.9 | 1.9 |
| Triethyl citrate NF | Plasticizer | 0.8 | 0.8 |
| Top coat | | | |
| Opadry® | Coating Agent | 2 | 2 |
| **Total Tablet Weight** | | **88.2** | **88.2** |

### Example 15. Color Coated Rasagiline Base Enteric Coated Formulation with Malic Acid

### Example 15a - 0.5 mg Rasagiline base

This example describes a 0.5 mg rasagiline base formulations containing malic acid with an extra color coating.

| **Component** | **Function** | **Per Tablet** **(mg)** |
|---|---|---|
| Core tablets | | |
| Rasagiline base | Drug Substance | 0.5 |
| Malic acid | Antioxidant | 1.6 |
| Mannitol | Filler | 80.3 |
| Aerosil | Flowing Agent | 0.6 |
| Starch, Pregelatinized (Starch STA-RX 1500) | Disintegrant | 20.0 |
| Starch NF | Binder | 10.0 |
| Talc | Lubricant | 2.0 |
| Stearic Acid | Lubricant | 2.0 |
| **Total Core Tablet Weight** | | **117.0** |
| Subcoating | | |
| Pharmacoat 606 (Hypromellose USP) Granules | Coating Agent | 4.8 |
| Purified Water | Processing Agent | |
| Coating Suspension | | |
| Eudragit L-30D-55 | Coating Agent | 6.25* |
| Talc USP Extra Fine | Lubricant | 3.1 |
| Triethyl citrate NF | Plasticizer | 1.25 |
| Purified Water | Processing Agent | |
| Top coat | | |
| **OPADRY II OY-GM-28900** **WHITE (catnum. 415850005)** **OR** **OPADRY II Y-30-18037** **WHITE (catnum. 415880719)** **OR/AND** **Opadry fx 63f97546silver** | Coating Agent | **1-5** |
| Purified Water | Processing Agent | |

| | | |
|---|---|---|
| * Dry substance remaining on the core. | | |

### Example 15b - 1.0 mg Rasagiline base

This example describes a 1 mg rasagiline base formulations containing malic acid with an extra color coating.

| **Component** | **Function** | **Per Tablet** **(mg)** |
|---|---|---|
| Core tablets | | |
| Rasagiline base | Drug Substance | 1.0 |
| Malic acid | Antioxidant | 1.6 |
| Mannitol | Filler | 79.8 |
| Aerosil | Flowing Agent | 0.6 |
| Starch, Pregelatinized (Starch STA-RX 1500) | Disintegrant | 20.0 |
| Starch NF | Binder | 10.0 |
| Talc | Lubricant | 2.0 |
| Stearic Acid | Lubricant | 2.0 |
| **Total Core Tablet Weight** | | **117.0** |
| Subcoating | | |
| Pharmacoat 606 (Hypromellose USP) Granules | Coating Agent | 4.8 |
| Purified Water | Processing Agent | |
| Coating Suspension | | |
| Eudragit L-30D-55 | Coating Agent | 6.25* |
| Talc USP Extra Fine | Lubricant | 3.1 |
| Triethyl citrate NF | Plasticizer | 1.25 |
| Purified Water | Processing Agent | |
| Top coat | | |
| **Opadry® II 31F20721 Blue** **OR** **Opadry® II 34G24627 Pink** **OR/AND** **Opadry fx 63f97546** **silver** | Coating Agent | **1-5** |
| Purified Water | Processing Agent | |

| | | |
|---|---|---|
| * Dry substance remaining on the core. | | |

### Example 16. Extraction of Rasagiline Base From Tablets

This example evaluated the amount of free Rasagiline base in 1 mg tablets of formulations with citric acid.

Rasagiline is assumed to be present in the formulation in salt form or as free base.

Rasagiline base is a non-polar compound very soluble in non-polar organic solvents such as hexane toluene and ethylacetate. Therefore, free Rasagiline base could be extracted from the solid formulation by these solvents.

Rasagiline salts are not soluble in non-polar solvents and the probability of the extraction of rasagiline citrate with hexane, toluene, 1-octanol or ethylacetate is very low.

Core tablets of Rasagiline base prepared using steps described in example 9 were tested. Each tablet contained 1 mg of Rasagiline base. Placebo tablets were used as references.

17 core tablets, 1 mg of Rasagiline base each were crushed and ground in mortar to homogeneous fine powder.

Each powder was mixed with 20 ml of organic solvent and stirred with magnetic stirrer for 1 hour at room temperature in closed glass vessel. Then the mixture was settled without stirring, the clear liquid was decanted and a sample of the resulting extract was filtered trough 0.2 µ filter.

The filtered samples of the extracts were subjected to HPLC analysis for quantity of dissolved Rasagiline. Samples of the placebo extracts were used as control.

Maximal possible calculated concentration of Rasagiline base in the extracts is 0.85 mg/ml (17 mg in 20 ml solvent).

The results are summarized in Table 5 below.

**Table 16. Extractions of Rasagiline base from core tablets with organic solvents**

| Experiment No. | No. of tablets | Weight of tablets, g | Solvent | Achieved concentration of Rasagiline in extract, mg/ml |
|---|---|---|---|---|
| 1 | 17 | 2.02 | Toluene | 0.01 |
| 2 | 17 | 2.02 | n-Hexane | 0.01 |
| 3 | 17 | 2.02 | DCM | 0.01 |
| 4 | 17 | 2.03 | 1-Octanol | 0.01 |
| 5 | 17 | 2.02 | Ethyl acetate | 0.02 |

### Summary of Results

The experimental results in Table 16 show that the core tablets of "Citric" formulation of Rasagiline base may contain 1 to 2 percent of the free rasagiline base extractable with non polar solvents.

Amount of the extractable base does not depend on the solvent type for non polar solvent as n-hexane, toluene, 1-octanol and dichloromethane. However, more polar solvent such as ethylacetate extracted more rasagiline base from the core tablets.

### Example 17. Clinical Study Based on Tablets According to Examples 3a and 3b

This study evaluated the bioavailability of two different rasagiline base 1 mg enteric coated tablet formulations prepared according to each of Examples 3a (Formulation I) and 3b (Formulation III) verses the marketed rasagiline drug product (Azilect® 1 mg) following a single dose administration, and to assess the effect of food on each one of the test formulations.

This study also evaluated the safety and tolerability of each treatment.

### 1. STUDY DESIGN

This study was a flexible two-part protocol, each part testing the bioavailability of a different rasagiline base 1 mg enteric coated formulation (Formulation I or Formulation III) against the reference product (Azilect® 1 mg).

Each part was an open-label, three-period, three-sequence, comparative crossover study in 15 healthy males and females (5 per sequence).
Treatment A: One Rasagiline Base 1 mg Enteric Coated Tablets (test Formulation I or test Formulation III) in the fasted state.
Treatment B: One Azilect® tablet (reference 1 mg rasagiline as rasagiline mesylate) in the fasted state.
Treatment C: One Rasagiline Base 1 mg Enteric Coated Tablets (test Formulation I or test Formulation III) following a standardized high-fat, high-calorie meal.

The 3 treatments were administered across 3 study periods each of which was separated by a 14-day washout interval.

Subjects were administered according to one of three sequences to which they were randomly assigned: A-B-C, B-C-A, or C-A-B.

In each period, subjects were confined for two overnight stays [at least 10.5 hours prior to and until at dose administration]. Subjects returned for an ambulatory blood sample collection (36 hours) on Day 2.

In Part 1, Subjects 1-15 received test Formulation I or reference, while in Part 2, Subjects 16-30 received test Formulation III or reference. The decision to proceed with each study part was based on the availability of the test Formulation.

AEs, vital signs, physical examination, and clinical laboratory tests were assessed for safety and blood samples were taken at regular pre-defined time points throughout the study for the measurement of rasagiline and aminoindan concentrations in plasma.

### 2. SUBJECT SELECTION

Thirty (30) healthy adult (∼50%/50% male and female) subjects were selected from non-institutionalized subjects consisting of members of the community at large.

### 3. PHARMACOKINETIC (PK) SAMPLING AND ANALYSIS

A total of 80 samples (about 400 mL) were drawn from each subject for PK purposes. Pharmacokinetic sampling occurs at the following timepoints:
a) Treatment A (test, fasted):
   Day 1 within 90 minutes prior to dosing (0 hour) and after dose administration at 0.5, 0.75, 1, 1.33, 1.67, 2, 2.33, 2.67, 3, 3.33, 3.67, 4, 4.5, 5, 6, 7, 8, 9, 12, 24 and 36 hours (22 samples).
b) Treatment B (reference, fasted):
   Day 1 within 90 minutes prior to dosing (0 hour) and after dose administration at 0.25, 0.5, 0.75, 1, 1.25, 1.5, 2, 3, 4, 5, 6, 7, 8, 12, 24 and 36 hours (17 samples).
c) Treatment C (test, fed):
   Day 1 within 90 minutes prior to dosing (0 hour) and after dose administration at 1, 1.5, 2, 2.5, 3, 3.33, 3.67, 4, 4.33, 4.67, 5, 5.33, 5.67, 6, 6.33, 6.67, 7, 7.33, 7.67, 8, 8.5, 9, 10, 11, 12, 13, 14, 15, 16, 18, 19, 20, 21, 22, 23, 24, 25, 26 and 36 hours (41 samples).

Blood was drawn either by direct venipuncture or through an indwelling intravenous cannula. Whenever the latter was performed, the cannula was flushed with 1.5 mL normal saline after each sampling. In addition, to avoid sample dilution, 1 mL blood was discarded before the next sample (as long as the cannula was in place). Therefore, up to 5 mL blood was collected at each time point. The total blood volume taken per subject for pharmacokinetic sampling was approximately 400 ml over a 4-week period.

Samples were collected into appropriate volume K2-EDTA vacutainers. The labels for all biological sample collection and storage containers contained, at a minimum, Protocol Number, Sub-study number, Subject Number; Dosing Period; Dosing Day; PK time point. Immediately following sample collection, samples were mixed by inverting the collection tube at least 2-3 times. Samples were cooled by an ice bath or cooling device until processed. Blood processing occurred within 2 hours of collection: the sample was centrifuged at approximately 2000 g and 4°C (± 3°C) for about 10 minutes, the plasma transferred into appropriately labeled duplicate polypropylene tubes, and stored at approximately -20°C until transfer or shipment to the bioanalytieal laboratory. At least 0.7 mL of plasma was transferred into the first polypropylene tube and the remaining plasma was transferred to the second polypropylene tube. The time at which samples were placed at -20°C were recorded in the study documentation.

Actual sampling time was recorded directly in the source data or CRF. Sample processing procedures were documented in the PK logbook.

The rasagiline and aminoindan plasma concentrations were measured using a validated LC/MS/MS bioanalytical method and according to the Bioanalytical Laboratory's Standard Operating Procedures and FDA Guidelines.

Analysis of the PK data of each sub-study was performed separately, according to audited bioanalytical data availability. The individual plasma concentrations of rasagiline and aminoindan were listed, displayed graphically as appropriate and summarized using descriptive statistics for each of the treatments.

Pharmacokinetic analysis were performed with rasagiline and aminoindan concentration profiles using appropriate non-compartmental methods.

The following parameters were calculated: Cₘₐₓₜ tₘₐₓ, t_{lag}, AUCₜ, AUC_{∞}, t_{1/2}, CL/F, V/F, %AUCₑₓₜ, regression coefficient of the terminal slope. Additional PK parameters were calculated if deemed necessary. All the PK parameters were listed and summarized using descriptive statistics.

Statistical analysis was performed using SAS for each substudy based on the reception of the data. For each sub-study, bioequivalence between the test and reference formulations in the fasted state and the food effect on the test formulation were evaluated only for rasagiline, according to 90% confidence intervals (CIs) of ratios of geometric means for Cₘₐₓ, AUCₜ, and AUCoo. The ratios and CI were calculated using ANCOVA on the log-transformed data (MIXED procedure, SAS). The conclusion regarding bioequivalence were based on the back-transformed point estimate and CI. Tₘₐₓ were analyzed using nonparametric analysis (Wilcoxon Signed Rank Test).

### 4. RESULTS

Tables 17a-17d below summarize testing results of this study.

### Bioequivalence tests

The testing results showed that the delayed release formulations tested (Formulation I and Formulation III) met the criteria for bioequivalence to the known immediate release formulation. Each of the Cₘₐₓ and AUCₜ achieved a range of 80-140% within a 90% confidence interval between the formulation tested and the reference immediate release formulation.

### MAO assay:

The testing results showed MAO-B activity for formulation prepared according to each of the Examples 3a and 3b were comparable to the reference immediate release formulation.

The standard method was used for the enzymatic determination of MAO: "Determination of monoamine oxidase (MAO) by an extraction method using radiolabelled substrate in various tissues".

Briefly, fifty (50) µl of homogenate were added to 100 µl 0.1 M phosphate buffer (pH-7.4). After preincubation of 20 minutes at 37°C, 50 µl of ¹⁴C-phenylethylamine hydrochloride (10 µM final concentration) were added and incubation continued for next 20 minutes. The reaction was then stopped by addition of citric acid 2 M.

Radioactive metabolites were extracted into toluene/ethyl acetate (1:1 v/v.), a solution of 2,5-diphenyloxazole was added to a final concentration of 0.4 % and the metabolite content is estimated by liquid scintillation counting.

Activity of rat brain homogenate served as standard (positive control) to the assay.

Protein determination was performed by the Lowrey method.

### Safety and tolerability

The testing results showed that safety and tolerability for each treatment were acceptable.

**Table 17a. PK Parameters in the Fasted State:**

| Study | Formulation | | t1/2 (h) | tmax (h) | Cmax (ng/mL) | AUC_{inf} (ng h/mL) |
|---|---|---|---|---|---|---|
| **Part 1** | **AZILECT®** | **Mean± SD** | **1.92 ±1.19** | **0.50±0.24** | **5.790±2.731** | **4.281±1.280** |
| | **Formulation I** | **Mean± SD** | **2.25±1.15** | **2.68±0.80** | **5.078±2.160** | **4.092±1.187** |
| **Part 2** | **AZILECT®** | **Mean± SD** | **2.65±3.31** | **0.50±0.21** | **6.22±2.585** | **4.960±1.807** |
| | **Formulation III** | **Mean± SD** | **2.12±0.78** | **2.33±0.80** | **5.739±1.406** | **5.073±1.045** |

**Table 17b. Bioequivalence in the Fasted State:**

| **Study** | **Formulation** | | **Single dose fasted** | |
|---|---|---|---|---|
| | | | **Point estimate** | **90% CI** |
| **Part 1** | **Formulation I** | **AUC** | **96** | **79 -117** |
| | | **Cmax** | **88** | **58 -133** |
| **Part 2** | **Formulation III** | **AUC** | **105** | **106 -117** |
| | | **Cmax** | **99** | **75 -130** |

**Table 17c. PK Parameters in the Fed State:**

| Study | Formulation | | t1/2 (h) | tlag (h) | tmax (h) | Cmax (ng/mL) | AUC_{inf} (ng h/mL) |
|---|---|---|---|---|---|---|---|
| **Part 1** | **Formulation I-Fast** | **Mean± SD** | **2.25**±**1.15** | **1.19±0.82** | **2.68±0.80** | **5.078±2.160** | **4.092±1.187** |
| | **Formulation I-Fed** | **Mean± SD** | **2.29**±**1.28** | **11.14±5.88** | **12.31±6.53** | **3.696±2.670** | **3.664±1.940** |
| **Part 2** | **Formulation III - Fast** | **Mean± SD** | **2.12**±**0.78** | **1.33±0.61** | **2.33±0.80** | **5.739±1.406** | **5.073±1.045** |
| | **Formulation III - Fed** | **Mean± SD** | **2.90**±**2.50** | **5.57±1.98** | **6.12±2.11** | **7.511±3.448** | **4.995±1.710** |

**Table 17d. Bioequivalence in the Fed State**

| **Formulation** | | **Single dose fed** | |
|---|---|---|---|
| | | **Point estimate** | **90% CI** |
| **Azilect®** | **AUC** | **81** | **71-85** |
| | **Cmax** | **49** | **29-50** |
| **Rasagiline Base** **Formulation I EC** | **AUC** | **82** | **68-100** |
| | **Cmax** | **58** | **38-87** |
| **Rasagiline Base** **Formulation III EC** | **AUC** | **95** | **79-113** |
| | **Cmax** | **121** | **90-164** |

### Conclusions:

As shown in Tables 17a-17d, Formulation III meets the requirements in both fasted and fed states. The PK parameters and bioequivalence of Formulation III are similar to those of Azilect^{®}.

### Example 18. Preparation of Rasagiline Citrate

Solid crystalline rasagiline base used in this example was prepared in a similar process as described below:

### A) Preparation of rasagiline base oil

100.0g of Rasagiline Tartrate was suspended in 458 ml deionized water, 229ml Toluene was added and 46 ml of 25% NaOH solution was introduced at stirring. The mixture was heated to 45°C, stirred at 45C for 15 minutes and settled at this temperature.

Two phases were separated. The lower aqueous phase (pH=13-14) was discarded, the upper toluenic phase was washed with 140 ml deionized water. The resulting emulsion was settled, and two phases were separated. The lower aqueous phase (pH=9-10) was discarded. The toluenic solution was evaporated under vacuum in evaporator.

After the solvent evaporation completion 60 ml isopropanol was added to the residue and evaporation was continued. After completion of the evaporation 50 ml of isopropanol was added and distilled out under the same conditions. The residue, oil of R-PAI base, was obtained.

### B) Crystallization of rasagiline base

The rasagiline base oil obtained in step A) above was dissolved in 56 ml isopropanol. The solution was cooled to 16°C and 147.5 ml of deionized water was added by portions in 3hr at cooling and stirring. During the addition of water precipitation development was observed and the batch was immediately seeded with crystalline R-PAI base.

The resulting suspension was cooled to 2°C, stirred at this temperature overnight and filtered. The solid was washed with water and dried at room temperature under vacuum. Solid dry R-PAI base were obtained, with a yield of 96% relative to oil base.

This example describes the preparation and characterization of rasagiline citrate salt. Rasagiline citrate is an attractive drug substance. Since citric acid is a tribasic compound, there are three possible forms of rasagiline citrate: mono-, di- and tri-citrate. Therefore, Rasagiline citrate described herein can be mono-rasagiline citrate, di-rasagiline citrate, or tri-rasagiline citrate, or a mixture thereof.

Because rasagiline is a weak base and pKₐ values of citric acid are 3.13, 4.76 and 6.40, it can be assumed that bonding of 1^{st} and 2^{nd} rasagiline base molecules to citrate is much more probable than bonding of the 3^{rd} rasagiline base molecule.

### Starting Materials

Citric acid - anhydrous acid of USP grade was used for preparation of citrate salts.

Rasagiline base - crystalline Rasagiline base prepared as described above in this example.

### Example 18a - Preparation of Rasagiline Citrate in ethanol-acetone

3.02g of Citric acid was dissolved in 10ml absolute ethanol at room temperature. 5.38g rasagiline base was dissolved in 15 ml absolute ethanol. Solution of rasagiline base was introduced by portions into the solution of citric acid under stirring. Significant exothermal effect was recorded during the addition, during which the solution temperature rose from 17° to 24°C during 10 minutes of addition. The resulting clear solution was stored in freezer at -18°C and no precipitation was observed.

Additional 2.71g of solid Rasagiline base was added to the above resulting clear solution. After prolonged stirring at 20-23°C the solid was dissolved and a viscous clear solution was obtained. The resulting viscous clear solution was stored overnight in freezer at -18°C. No solid precipitation from the solution was observed during 20 hrs in the freezer.

The solution was evaporated under vacuum on rotary evaporator, the resulted residue (11.2g) of honey-like semi-solid material was held over weekend in freezer (-18°C). No crystallization of solid was observed.

The semi-solid material was mixed with 40ml acetone at stirring, no dissolution of the semi-solid material was observed during prolonged stirring.

Absolute ethanol (3ml) was then added to the mixture by portions at stirring. Complete dissolution of the semi-solid material was observed, the resulting clear solution was held overnight in freezer.

Honey-like semi-solid material precipitated from the acetone-ethanol solution was found on the bottom of the flask. The solution was decanted and the precipitate was dried under vacuum (20 mbar) for 4 hours. During the drying a stabile foam formed. The flask with the foam was connected to high vacuum pump and dried at 2-3 mbar overnight.

The foam solidified under high vacuum. The vacuum was disconnected and the material was broken up with spatula. 6.1g of white powder was obtained.

### Analysis:

Assay of Rasagiline base by HPLC - 60.8%
Crystallinity by XRD - Amorphous
Thermal analysis:
DSC - Peak at 179.7°C (128 exo), TGA - LOD=1.2% (25-100°C),
cont. weight loss at T>100°C.

### Example 18b - Mono-Citrate salt in water-acetone (molar ratio 1:1)

3.02g of Citric acid was dissolved in 4 ml deionized water. 2.69g of Rasagiline base was added to the solution. Exothermic effect was observed (temperature rose from 22 to 25°C), and most of the solid was dissolved. Then the mixture was heated to 42° and complete dissolution of the solid was observed. The resulting clear viscous syrup-like solution was held in refrigerator at +5°C overnight. No precipitation was observed during 15 hrs.

The solution was mixed with 15 ml acetone and evaporated on rotary evaporator under vacuum. The residue of honey-like semi-solid material (6.29g) was dried under vacuum (20 mbar) at ambient temperature. A foam formed (6.11g) during the drying and then further dried under high vacuum (2-3 mbar).

The foam was solidified under high vacuum. The vacuum was disconnected and the material was broken up with spatula. 5.58g of white powder was obtained.

### Analysis:

Assay of Rasagiline base by HPLC - 44.5%
Crystallinity by XRD - Amorphous
Thermal analysis:
DSC - Peak at 188.6°C (61 exo), TGA - LOD=1.5% (25-100°C),
cont. weight loss at T>100°C.

### Example 18c - Di-Citrate salt in water-acetone (molar ratio 2:1)

3.45g of Citric acid was dissolved in 5 ml deionized water and pre-heated to 30°C. 6.13g of Rasagiline base was added to the solution. Exothermic effect was observed (temperature rose from 30 to 36°C), and the solid was dissolved. The resulted clear viscous syrup-like solution was held in refrigerator at +5°C overnight. No precipitation was observed during 15 hrs.

The solution was mixed with 18 ml acetone and evaporated on rotary evaporator under vacuum. The residue of honey-like semi-solid material (9.7g) was dried under vacuum (20 mbar) at ambient temperature. A foam formed during the drying and then further dried under high vacuum (2-3 mbar).

The foam was solidified under high vacuum. The vacuum was disconnected and the material was broken up with spatula. 8.81g of white powder was obtained.

### Analysis:

Assay of Rasagiline base by HPLC - 60.9%
Crystallinity by XRD - Amorphous
Thermal analysis:
DSC - Peak at 180.2°C (141 exo), TGA - LOD=1.2% (25-100°C),
cont. weight loss at T>100°C.

### Example 18d - Tri-Citrate salt in water-acetone (molar ratio 3:1)

3.46g of Citric acid was dissolved in 5 ml deionized water. 9.19g of Rasagiline base was added to the solution. Exothermic effect was observed (temperature rose from 22 to 27°C), and most of the solid was dissolved. Then the mixture was heated to 46°C 0.5ml water was added and complete dissolution of the solid was observed. The resulting clear viscous syrup-like solution was held in refrigerator at +5°C overnight. No precipitation was observed during 15 hrs.

The solution was mixed with 18 ml acetone and evaporated on rotary evaporator under vacuum. The residue of honey-like semi-solid material (13.20g) was dried under vacuum (20 mbar) at ambient temperature. A foam formed during the drying (13.19g) and then further dried under high vacuum (2-3 mbar).

The foam was solidified under high vacuum. The vacuum was disconnected and the material was broken up with spatula.
12.80g of white powder was obtained.

### Analysis:

Assay of Rasagiline base by HPLC - 70.6%
Crystallinity by XRD - Amorphous
Thermal analysis:
DSC - Peak at 181.8°C (136 exo), TGA - LOD=1.3% (25-100°C),
weight loss at T>100°C.

### Discussion of Example 18

Experimental observations show exothermic reactions between Rasagiline base and Citric acid in aqueous solutions. The fact that Rasagiline base with aqueous solubility of about 2 mg/ml dissolves in aqueous reaction solution at more than 10 wt% demonstrates complete or near-complete conversion of the base into salt.

At the same time a fraction of the base could be extracted from the salt solution with non-polar organic solvent as toluene.

The preparation of mono-, di- and tri- citrate salts of rasagiline can be calculated from molecular weights of Rasagiline (R-PAI), Citric acid and water. The calculation results are presented in Table 18 below. The data presented in Table 18 also demonstrate that R-PAI content in the citrates prepared in the examples 18a-18d conforms to the composition of hydrate salts.

**Table 18. Rasagiline Citrates calculated composition**

| Salt | Composition | MW | R-PAI content %wt. | Water content %wt. |
|---|---|---|---|---|
| Mono-Citrate | (R-PAIH⁺) CitH₂⁻ | 363.3 | 47.1 | 0 |
| Di-Citrate | (R-PAIH⁺)₂CitH⁻² | 534.5 | 64.0 | 0 |
| Tri-Citrate | (R-PAIH⁺)₃Cit⁻³ | 705.7 | 72.8 | 0 |
| Mono-Citrate monohydrate | (R-PAIH⁺) CitH₂⁻·H₂O | 381.3 | 44.9 | 4.7 |
| Di-Citrate monohydrate | (R-PAIH⁺)₂CitH⁻²·H₂O | 552.5 | 61.9 | 3.2 |
| Tri-Citrate monohydrate | (R-PAIH⁺)₃Cit⁻³·H₂O | 723.7 | 70.9 | 2.5 |
| Di-Citrate dihydrate | (R-PAIH⁺)₂CitH⁻²·2H₂O | 570.5 | 60.0 | 3.1 |

| | | | | |
|---|---|---|---|---|
| R-PAIH⁺- Rasagiline base (R-PAI) cation Cit⁻ⁿ - Citrate anion | | | | |

Rasagiline citrate salts prepared in Examples 18a-18d demonstrate extremely high aqueous solubility. Solutions of mono- di- and tricitrate salts prepared in the Examples 18b, 18c and 18d had concentrations of dissolved solid of 59, 66 and 70 wt%, respectively. These solutions did not show saturation and were found stable at low temperatures. No precipitation was observed during 15 hrs at +5°C. This data shows extremely high solubility of the citrate salts of rasagiline in water. Solutions with more than 70% wt of rasagiline citrate could be prepared. Rasagiline citrate salts having 3-10 wt% water content appear as syrups or honey-like semi-solid.

The most soluble rasagiline salt described previously is monobasic maleate salt of rasagiline, which has a solubility not less than 1000 mg/ml water, as described in U.S. Patent No. 6,630,514. But the phenomenon of extremely high solubility exhibited by rasagiline citrate was not observed in any previously identified salt of rasagiline.

Such extremely high solubility is a property of practical value, and allows for preparation of highly concentrated liquid and semi-solid formulations. Aqueous or alcoholic solution of Rasagiline Citrate containing 60-80% of active pharmaceutical ingredient (API) could be used in the production of transdermal patches, sublingual strips, and other formulations benefiting from such highly concentrated liquid or semi-solid. Such highly concentrated solutions are also useful for optimizing the efficiency of production processes, e.g. for tablets.

### Example 19. Additional Preparation of Rasagiline Citrate

Solid crystalline rasagiline base used in this example was prepared in a similar process as described below:

### A) Preparation of rasagiline base oil

100.0g of Rasagiline Tartrate was suspended in 458 ml deionized water, 229ml Toluene was added and 46 ml of 25% NaOH solution was introduced at stirring. The mixture was heated to 45°C, stirred at 45C for 15 minutes and settled at this temperature.

Two phases were separated. The lower aqueous phase (pH=13-14) was discarded, the upper toluenic phase was washed with 140 ml deionized water. The resulting emulsion was settled, and two phases were separated. The lower aqueous phase (pH=9-10) was discarded. The toluenic solution was evaporated under vacuum in evaporator.

After the solvent evaporation completion 60 ml isopropanol was added to the residue and evaporation was continued. After completion of the evaporation 50 ml of isopropanol was added and distilled out under the same conditions. The residue, oil of R-PAI base, was obtained.

### B) Crystallization of rasagiline base

The rasagiline base oil obtained in step A) above was dissolved in 56 ml isopropanol. The solution was cooled to 16°C and 147.5 ml of deionized water was added by portions in 3hr at cooling and stirring. During the addition of water precipitation development was observed and the batch was immediately seeded with crystalline R-PAI base.

The resulting suspension was cooled to 2°C, stirred at this temperature overnight and filtered. The solid was washed with water and dried at room temperature under vacuum. Solid dry R-PAI base were obtained, with a yield of 96% relative to oil base.

This example describes the additional preparations and characterization of rasagiline citrate salt.

### Starting Materials

Citric acid - anhydrous acid of USP grade was used for preparation of Citrate salts.

Rasagiline base - pure crystalline Rasagiline base (DS) prepared as described in example 18 was used in this study.

### Example 19.1

3.84g of citric acid was dissolved in 25ml water and 3.42g of rasagiline base was added to the solution, which was stirred at room temperature and monitored by TLC. After 30 minutes no traces of R-PAI was observed on TLC. The reaction mixture was extracted with 2x30 ml toluene after one hour. The combined toluenic extract was evaporated to dryness. Yield: 0.06g (1.75%) (R-PAI).

The aqueous phase was evaporated in vacuum to dryness. Hony-like semi-solid product was obtained. Yield: 7.53g (103.7%).

### Example 19.2

1.92g of citric acid was dissolved in 10ml water and 1.71g of rasagiline base added to the solution. The mixture was stirred for 18 hours and then the solvent was removed by lyophilization (1-0.3mbar; -20 - +20 °C; 46hours). Yield: 3.69g (101.65%).

The product was solid foam but after several hours became a semi-solid honey-like material. According to NMR data the salt formed with 0.73 equivalent of acid.

### Example 19.3

1.92g of citric acid was dissolved in 15ml water and 3.42g of rasagiline base was added to the solution. The reaction mixture was stirred at room temperature for 22 hours. The water was removed by lyophilization (1 - 0.3 mbar, -20 - +20°C; 46 hours).

Crystalline-like foam were obtained, which then became semi-solid honey-like material in a few hours. According to the data of NMR the salt formed with 0.48 equivalent of acid.

### Example 19.4

3.84g of citric acid was dissolved in 30ml water and 6.84g of rasagiline base was added to the solution, which was stirred for 2 hours and then the reaction mixture was extracted with 2x40ml toluene. The combined toluenic extract was evaporated to dryness. 20ml IPA was added to the residue and the solvent was evaporated in vacuum to dryness. Yield: 1.5g (22%, R-PAI).

The aqueous phase was evaporated to dryness, resulting honey-like semi-solid product. Yield: 9.47g (103.3%).

¹H-NMR - 0.65 equivalent of acid formed the salt.

### Example 19.5

3.84g of citric acid was dissolved in 50ml water and 10.26g of rasagiline base was added to the solution, which was stirred at room temperature for 3 hours.

The reaction mixture was extracted with 2x50 ml toluene. The combined toluenic extract was evaporated to dryness in vacuum.
IPA was added to the residue and then evaporated to dryness.
Yield: 3.92-4.13g (R-PAI) (38.2-40.2%).

The aqueous phase was evaporated to dryness, resulting honey-like semi-solid product. Yield: 10.54-9.73g.

¹H-NMR - 0.58 equivalent of acid formed the salt.

### Example 19.6

3.84g of citric acid was dissolved in 50ml water and 10.26g of rasagiline base was added to the solution, which was stirred for 3 hours at 60 °C.

The reaction mixture was extracted with 2x50 ml toluene. The combined toluenic extract was evaporated to dryness in vacuum.
IPA was added to the residue and then evaporated to dryness.
Yield: 3.92-4.13g (R-PAI) (38.2-40.2%).

The aqueous phase was evaporated to dryness, resulting honey-like semi-solid product. Yield: 10.54-9.73g.

¹H-NMR - 0.58 equivalent of acid formed the salt.

### Example 19.7

3.84g of citric acid was dissolved in 50ml water and 10.26g of rasagiline base was added to the solution, which was stirred for 42 hours at 25°C.

The reaction mixture was extracted with 2x50 ml toluene. The combined toluenic extract was evaporated to dryness in vacuum.
IPA was added to the residue and then evaporated to dryness.
Yield: 3.92-4.13g (R-PAI) (38.2-40.2%).

The aqueous phase was evaporated to dryness, resulting honey-like semi-solid product. Yield: 10.54-9.73g.

¹H-NMR - 0.58 equivalent of acid formed the salt.

### Example 19.8

1.92g of citric acid was dissolved in 25ml water and 5.13g of rasagiline base was added to the solution, which was stirred at room temperature for 16 hours. The reaction mixture was extracted with 2x30ml toluene and the toluenic extract was evaporated to dryness. Yield: 2.19g (R-PAI ;42.7%).

The aqueous phase was dried by lyophilization. The product was crystalline-like foam which then became honey-like semi-solid.

¹H-NMR - 0.55 equivalent of acid formed the salt.

### Example 19.9

1.92g of citric acid was dissolved in 25ml water and 5.13g of rasagiline base was added to the solution, which was stirred at room temperature for 9 days. The solid was filtered off, washed with 5ml water, and dried with air. Yield: 0.31g (6%, R-PAI), Mp.: 39.3-41.0 °C.

The aqueous phase was lyophilized. The crystalline-like foam was formed which became honey-like semi-solid in a few hours.

¹H-NMR - 0.35 equivalent of acid formed the salt.

### Example 19.10

1.6g of citric acid was dissolved in 10ml water and 1.0g of rasagiline base was added to the solution, which was stirred at room temperature. The solvent was removed by lyophilization. The product was crystalline-like foam which became semi-solid after a few hours.

¹H-NMR - 1.2 equivalent of acid formed the salt.

### Example 19.11

1.92g of citric acid was dissolved in 15ml IPA and 1.71g of rasagiline base was added to the solution, which was stirred at room temperature for 2 hrs. No R-PAI was detected by TLC.
The solvent was removed in vacuum. Yield: 3.85 (106%)

The foam-like semi-solid product became honey-like upon contacting with the humidity in the air.

### Example 19.12

1.92g of citric acid was dissolved in 15ml IPA and 3.42g of rasagiline base was added to the solution, which was stirred at room temperature for 2 hours. The reaction mixture became clear, which was monitored by TLC (hexane:EtOAc = 1:1). The traces of R-PAI was detected. The solvent was removed in vacuum. The residue was slurred in 2x30ml toluene. The combined toluenic phase was evaporated to dryness. Yield: 0.65g (19%; R-PAI).

The crude product was disolved in IPA and the solution was evaporated to dryness, resulting honey-like product.

### Example 19.13

1.92g of citric acid was dissolved in 15ml IPA and 5.13g of rasagiline base was added to the solution, which was stirred at room temperature for 2 hours. The reaction was monitored by TLC. Free R-PAI was present. The solvent was removed in vacuum. The residue was slurred in 2x30ml toluene. The combined toluenic phase was evaporated to dryness. Yield: 2.47g (48%; R-PAI).

The crude product was disolved in IPA and the solution was evaporated to dryness. A honey-like product was obtained.

### Example 19.14

1.92g of citric acid was dissolved in 15 ml methanol and 1.71 g of rasagiline base was added to the solution, which was stirred at room temperature for 22 hours and was then evaporated to dryness. Yield: 3.77g (103.86%).
¹H-NMR - 0.72 equivalent of acid formed the salt.

### Example 19.15

1.92g of citric acid was dissolved in 20 ml methanol and 3.42 g of rasagiline base was added to the solution, which was stirred at room temperature for 22 hours and was then evaporated to dryness. Yield: 5.48g-103.6%. By TLC, the free R-PAI in product was detected.
¹H-NMR - 0.5 equivalent of acid formed the salt.

### Example 19.16

1.92g of citric acid was dissolved in 25 ml methanol and 5.13g of rasagiline base was added to the solution, which was stirred at room temperature for 22 hours and was then evaporated to dryness. Yield: 7.32g-103.8%. By TLC, the free R-PAI in product was detected.
¹H-NMR - 0.33 equivalent of acid formed the salt.

### Example 19.17

1.92g of citric acid was stirred in 20ml EtOAc and 1.71g of rasagiline base was added to the solution, which was stirred for additional 72 hours. The reaction was monitored by TLC.
The free rasagiline base was detected.

The solution was decantated from the reaction mixture. The solvent was removed under vacuum. Yield: 1.32g (77%) R-PAI.

The isolated R-PAI was re-disolved in 20ml ethylacetate and and 10ml water was added to the mixture. The reaction mixture was stirred for 22 hours. The unreacted R-PAI remained in the EtOAc phase according to the data of TLC. The phases were separated. The organic phase was evaporated to dryness. Yield: 0.13g (7.6%) R-PAI.

### Example 19.18

1.92g of citric acid was stirred in 20ml EtOAc and 3.42g of rasagiline base was added. The solution was stirred for additional 72 hours. The reaction was monitored by TLC. The free rasagiline base was detected.

The solution was decantated from the reaction mixture. The solvent was removed under vacuum. Yield: 2.87g(83.9%, R-PAI).

The isolated R-PAI was re-dissolved in 20ml ethylacetate and 10ml water was added to the mixture. The reaction mixture was stirred for 22 hours. The unreacted R-PAI remained in the EtOAc phase according to the data of TLC. The phases were separated. The organic phase was evaporated to dryness. Yield: 0.62g (18%-R-PAI).

### Example 19.19

1.92g of citric acid was stirred in 25ml EtOAc and 5.13g of rasagiline base was added. The reaction mixture was stirred for additional 72 hours. The reaction was monitored by TLC.
The free rasagiline base was detected in all cases.

The solution was decantated from the reaction mixture. The solvent was removed in vacuum. Yield: 4.49g (87.5%, R-PAI).

The isolated R-PAI was re-disolved in 20ml ethylacetate and and 10ml water was added to the mixture. The reaction mixture was stirred for 22 hours. The unreacted R-PAI remained in the EtOAc phase according to the data of TLC. The phases were separated. The organic phase was evaporated to dryness. Yield: 1.76g (34.3% R-PAI).

### Example 19.20

1.92g of citric acid was stirred in 25ml toluene and 1.71g rasagiline base was added to the mixture. The heterogenous mixture was stirred at room temperature for 24 hours. The solution was decantated from the reaction mixture. The toluenic phase was evaporated to dryness. Yield: 1.58g (92.4%); (R-PAI by TLC).

The isolated R-PAI was re-dissolved in 10ml of toluene and was returned to the solid phase. 20ml water was added to the heterogenous mixture and stirred for 3 hours. The reaction was monitored by TLC. The phases were separated. The toluenic phase was evaporated to dryness. Yield: 0.12g (7%), R-PAI was detected according to the data of TLC. The aqueous phase was evaporated to dryness.

### Example 19.21

1.92g of citric acid was dissolved in 20ml acetone and 1.71g of rasagiline base was added to the reaction mixture, which was stirred at room temperature for 2 hours. The reaction was monitored by TLC. No R-PAI was detected.

The solution was decantated from the honey-like precipitation.
Yield: 2.43g (66.9%).

The acetonic solution was evaporated to dryness. Honey-like product was obtained. Yield: 1.48 (40.7%).

The total yield was 107.6% (acetone remained in the product).

### Example 19.22

1.92g of citric acid was dissolved in 20ml acetone and 3.42g of rasagiline base was added to the mixture, which was stirred at room temperature for 22 hours. R-PAI was detected by TLC.
The acetonic solution was decantated from the honey-like precipitation. Yield: 4.41g (82.6%) semi-solid product.

The acetonic phase evaporated to dryness. Yiels: 1.34g (25.1%)

**Table 19a. Summary of Experimental Results**

| Example | Solvent | Proportions of reagents (mole) | | Extracted R-PAI (%) (*Filtered) | Equivalent of Citric acid in the salt by NMR | R-PAI By TLC | pH (after extraction of R-PAI) |
|---|---|---|---|---|---|---|---|
| | | Citric acid, mole | Rasagiline base, mole | | | | |
| 19.11 | IPA | 1 | 1 | | | - | |
| 19.1 | Water | 1 | 1 | Extr. with Toluene 0.06g-1.75% | | - | |
| 19.4 | Water | 1 | 2 | 1.51g (22%) | | + | |
| 19.5 | Water | 1 | 3 | 4.13g (40.2%) | | + | |
| 19.6 | Water 60°C | 1 | 3 | 4.44g (43.3%) | | + | |
| 19.7 | Water 2 days | 1 | 3 | 3.92g (38.2%) | | + | |
| 19.9 | Water | 1 | 3 | 0.31g (6%) | 0.35 | + | |
| 19.3 | Water | 1 | 2 | | 0.48 | + | |
| 19.13 | IPA | 1 | 3 | 2.47g (48%) | 1.55 | + | |
| 19.12 | IPA | 1 | 2 | 0.65g (19%) | 1.62 | + | |
| 19.10 | Water | 1 | 0.7 | lyophilized | 1.2 | - | |
| 19.8 | Water | 1 | 3 | Extr.2.19g (42.7%) | | + | |
| 19.2 | Water | 1 | 1 | lyophilized | 0.73 | - | |
| 19.14 | MeOH | 1 | 1 | | 0.72 | - | 3,53 (3,49) |
| 19.15 | MEOH | 1 | 2 | | 0.5 | + | 4,88 (4.46) |
| 19.16 | MeOH | 1 | 3 | | 0.33 | + | 6,09 (4,66) |
| 19.20 | Toluene | 1 | 1 | | | + | |
| 19.17 | EtOAc | 1 | 1 | | 7.6 | + | (3.41) |
| 22.18 | EtOAc | 1 | 2 | | 18 | + | (4.43) |
| 19.19 | EtOAc | 1 | 3 | | 34.3 | + | (4.90) |
| 19.21 | Acetone | 1 | 1 | | | - | |
| 19.22 | Acetone | 1 | 2 | | | + | |

### Discussion of Example 19

Rasagiline base readily forms salts with citric acid in almost all various types of solvents, but most readily in water and in alcohols.

Mono-rasagiline citrate salt forms and is stable in most solvents. A few percent of free rasagiline may be extracted from the aqueous solution of this salt.

Di- and tri-citrates are not as stable in the aqueous and other solutions (alcohol, MEK, acetone). Free rasagiline base may be detected by TLC and extracted with toluene.

The separation of free rasagiline base from the aqueous solution of di- and tri-rasagiline citrates resulted the change of pH of the solution, as shown in Table 19a.

All of the rasagiline citrate salts are hygroscopic salts and readily absorb the humidity from air. The rasagiline citrates more readily form strong solvates with the solvents in which the salt formation occured (up to 10%).

The aqueous solution of rasagiline citrates may be dryed by lyophilization.

The NMR study of rasagiline citrate in the above examples provides information about the composition (proportion) of the samples and not the proportion of the free base and the charged base (cationic form) with citric acid.

The results of this example also demonstrate that the ratio of rasagiline base:citric acid used correlates with content of the "extractable" rasagiline base and amount of unreacted Citric acid found in the salt by NMR. The results are summarized in the Table 19b below.

**Table 19b. Effect of salt composition on content of extractable rasagiline base**

| Salt | Ratio Base to acid | Equivalent of Citric acid by ¹HNMR for salt prepared in: | | Content of extractable base (toluene) |
|---|---|---|---|---|
| | mole:mole | Methanol | Water | % on total base content |
| Mono citrate | 0.7:1.0 | N.A. | N.A. | N.A. |
| Mono citrate | 1.0:1.0 | 0.72 | 0.73 | 1.75 - 3.6 |
| Di citrate | 2.0:1.0 | 0.50 | 0.48 | 22.0 |
| Tri citrate | 3.0:1.0 | 0.33 | 0.35 | 42.7 |

The data in Table 19b show that excess of citric acid dramatically reduce the content of extractable rasagiline base.

It is concluded that lower content of extractable rasagiline base (or higher content of citric acid) provides higher stability of Rasagiline in the salt. Therefore, the most stable rasagiline citrate salt is mono- citrate salt and the most stable compositions of rasagiline citrate are compositions containing less than 1 mole of Rasagiline base per 1 mole of Citric acid.

### Example 20. Evaluation of Rasagiline Citrate salts

Three samples of rasagiline citrates prepared in examples 18b, 18c and 18d were exposed to atmospheric air in open dishes at ambient temperature. The changes were observed and recorded. The results are presented in the Table 20a below:

**Table 20a. Changes in Citrate salts exposed to atmosphere at ambient temperature**

| | Example 4b | Example 4c | Example 4d |
|---|---|---|---|
| Salt type | Mono- | Di- | Tri- |
| Time of exposure (hrs:min) | | | |
| 0:00 | Powder | Powder | Powder |
| 0:30 | | Powder | Powder |
| 0:50-1:00 | Sticky aggregates | Powder | Sticky aggregates |
| 1:50-2:00 | Semi-solid | Powder | Sticky aggregates |
| 5:00 | Honey-like semi-solid | Lump powder | Semi-solid |
| 6:00 | Syrup | Sticky aggregates | Semi-solid |
| 7:00 | Syrup | Sticky aggregates | Honey-like semi-solid |
| 25:00 | N.A. | Sticky aggregates + semi-solid | N.A. |

### Discussion

The results in Table 20a show that all three salts disclosed above are highly hygroscopic when exposed to atmosphere at ambient temperature. The results also show that there is no significant difference in hygroscopicity between the mono-, di- and tri- rasagiline citrates. All three salts appear as hydrates.

Parkinsonian patients suffer from swallowing disorders which prevent them from swallowing standard tablets or capsules. (Potulska A., "Swallowing disorders in Parkinson's disease", Parkinsonism Relat. Disord. (2003 Aug) Vol. 9(6), pages 349-53). This difficulty hinders their treatment by reducing patient compliance. Patients will be more likely to comply to dosage regimens if swallowing tablets or capsules is not required.

A means to avoid the absorption of rasagiline in the stomach, and to eliminate the need for swallowing tablets, is by absorption of rasagiline into the body before reaching the stomach. Such absorption of rasagiline, and hence resolution of both problems, can be accomplished by contact with the buccal, sublingual, pharyngeal and/or esophageal mucous membranes. To accomplish this, oral compositions can be designed to rapidly disperse within the mouth to allow maximum contact of rasagiline with the buccal, sublingual, pharyngeal and/or esophageal mucous membranes. The unexpectedly high hygroscopicity of the citrate salts of rasagiline is particularly suitable for such oral formulations.

Another three samples of rasagiline citrates prepared in Examples 18b, 18c and 18d were stored in closed transparent glass vials sealed with paraffin film in refrigerator at 7±2°C. The changes were observed and recorded. The results are presented in the Table 20b below:

**Table 20b. Appearance of Citrate salts stored in refrigerator**

| | Example 4b | Example 4c | Example 4d |
|---|---|---|---|
| Salt type | Monobasic | Dibasic | Tribasic |
| Time of Storage, month(s) | Appearance: | | |
| 0 | White powder | White powder | White powder |
| 3 | White powder | White powder | White powder |
| 6 | White powder | White powder | White powder |

### Discussion

The results in Table 20b show that all three salts could be stored for a long time (more than 6 month) under sealed condition at low temperature (∼7°C) with no change of color and appearance in spite of their high hygroscopicity at ambient temperature. This finding was surprising and may be the result of effect of temperature on hygroscopic point of Rasagiline citrates.

The results in Table 20b also show that all three salts could be handled and processed under controlled conditions such as low temperature and low humidity without change of their physical appearances, despite their high hygroscopicity.

### Example 21. Characterization of Rasagiline Citrate - XRD Analysis

Samples were tested using Scintag X-Ray powder diffractometer model X'TRA, Cu-tube, solid-state detector.
Scanning parameters
Range: 2-40 degrees two-theta.
Scan mode: Continuous scan
Step size: 0.05 deg.
Rate: 3 deg./min.
SAMPLE HOLDER: A ROUND STANDARD ALUMINUM SAMPLE HOLDER WITH ROUND ZERO BACKGROUND QUARTZ PLATE WITH CAVITY OF 25 (DIAMETER) *0.5 (DEPT.) MM.

**TABLE 21. CHARACTERISTIC XRD PEAK POSITIONS OF THE DIFFERENT SAMPLES (± 0.2 DEGREES TWO-THETA)**

| Form | Amorphous form | Amorphous form | Amorphous form | Amorphous form |
|---|---|---|---|---|
| Sample | 1 | 2 | 3 | 4 |
| Peak positions | Not applicable | Not applicable | Not applicable | Not applicable |

### Discussions

Results in Table 21 show that samples of rasagiline citrate do not show any characteristic peaks in XRD analysis, which indicates that rasagiline citrates prepared are of the form of amorphous.

### Example 22. Comparison of Properties of Rasagiline Citrate to Other Salts

Rasagiline citrate exhibits properties which are different from the properties of other citrate salts as shown in Table 22a, and also different from the properties of other rasagiline salts as shown in Table 22c.

**Table 22a - Summary of Citrate of various drug substances**

| **Citrate Salt of Drug Substance** | **Polymorph** | **References** |
|---|---|---|
| 5,8,14-Triazatetracyclo-hexdecqa-2(11),3,5,7,9-pentaene | Crystalline | WO 02/092597 |
| 2-hydroxy-3-[5-(morpholin-4-ylmethol)pyridine-2-yl]1H-indole-5-carbonitrile | Crystalline | WO 07/089191 |
| 2-(6-{2-[(2(2R)-2-Methyl-1-pyrrolidin-1-yl]-ethyl}-2-naphthalen-2-yl)-2H-pyridazin-3-one | Crystalline | US 2005/0256127 |
| 4-(3,4-dichlorophenyl)-2-[2-94-Methlpiperazin-1-yl)-Bennylidene]-thiomorpholin-3-one | Crystalline | US 2003/0181444 |
| 4-[(4-Methyl-1-piperazinyl)methyl]-N-[4-methyl-3-[[(4-(3-pyridinyl)-2-pyrimidinyl]amino]phenyl]-benzamide | Crystalline | US 2008/0249104 |
| 5-(5-Fluoro-2-oxo-1,2-dihydro-indol-3-ylidenemethyl)-2,4-dimethyl-1H-pyrrole-3-carboxylic acid (2-pyrrolidin-1-yl-ethyl)-amide | Crystalline | US 2008/0275101 |
| 3-{(3R,4R)-4-methyl-3-[methyl-(7H-pyrrole]2,3-d]pyrimidin-4-yl)-amino]-piperidin-1-yl}-3-oxo-propionitrile | Crystalline | US 2005/0159434 |
| Decitabine | Crystalline | US 2006/0069060 |

As shown in Table 22a, unlike citrate salts of other drug substance, the rasagiline citrate salts are amorphous. No crystalline forms of rasagiline citrate have been detected.

**Table 22b - Summary of Properties of Rasagiline Base and Citric Acid**

| | Hygroscopicity by KF | Water solubility (mg/ml) | References |
|---|---|---|---|
| Solid R-PAI (Free Base) | Not hygroscopic | low | US 2008/0161408 |
| Citric Acid | low | 1330 | "Pharmaceutical Excipients" database |

**Table 22c - Summary of Properties of Rasagiline Salts**

| R-PAI base/ R-PAI salts | Hygroscopicity by KF | Water solubility (mg/ml) | References |
|---|---|---|---|
| Chloride | Not hygroscopic | 238 | US 5,457,133 |
| Mesylate | Not hygroscopic | 635 | US 5,532,415 |
| Tartrate | Not hygroscopic | 33 | US 5,532,415 |
| Maleate | N.A. | >=1000 | US 5,532,415 |
| Sulphate | N.A. | 485 | US 5,532,415 |
| Tosylate | N.A. | 60-70 | US 5,532,415 |
| Fumarate | N.A. | 95 | US 5,532,415 |
| Phosphate | N.A. | >=720 | US 5,532,415 |
| Esylate | N.A. | >=300 | US 5,532,415 |
| Acetate | N.A. | >=720 | US 5,532,415 |
| Tannate | <10% (R-PAI content related) | low | US 7,547,806 |
| Citrate | Highly hygroscopic | Extremely High (Higher than rasagiline maleate) | |
| Edisilate | Not hygroscopic | 342.5 | WO 2008/019871 |
| Oxalate | Not hygroscopic | 19.7 | WO 2008/019871 |

The results in Table 22b and 22c show that compared to rasagiline base and other rasagiline salts, rasagiline citrate salt exhibits the highest water solubility and highest hygroscopicity.

## Claims

1. A stable oral dosage form comprising a core having a production process-resulting form of rasagiline base and at least one pharmaceutically acceptable excipient; and an acid resistant pharmaceutically acceptable coating, the production process comprising
a) preparing the core by admixing rasagiline base, citric acid and/or malic acid, and a pharmaceutically acceptable excipient; and
b) coating the core with the acid resistant pharmaceutically acceptable coating.

2. The dosage form of claim 1, wherein step a) comprises preparing a wet granulate of the rasagiline base and citric acid, and a pharmaceutically acceptable excipient, and wherein step a) further comprises:
i) drying the wet granulate to form a dry granulate, preferably in a fluid bed dryer under inlet air temperature of 40°C to 50°C, and under outlet air temperature of not greater than 37°C,
ii) milling the dry granulate to form particles, preferably through an oscillating granulator, and
iii) admixing the particles with at least one lubricant, which lubricant is preferably talc or stearic acid, or a combination thereof.

3. A stable oral dosage form comprising a core having at least one pharmaceutically acceptable excipient and rasagiline base, rasagiline citrate, rasagiline malate, or a mixture of at least two of rasagiline base, rasagiline citrate, and rasagiline malate; and an acid resistant pharmaceutically acceptable coating.

4. The stable oral dosage form of claim 3, which is less than 150 mg by weight, wherein the core consists essentially of at least one pharmaceutically acceptable excipient and rasagiline citrate, wherein the at least one pharmaceutically acceptable excipient in the core is at least one anti-oxidant and at least one disintegrant,
wherein the anti-oxidant is preferably citric acid and the disintegrant is preferably present in the core at an amount between 0.5% and 20% by weight, and wherein the disintegrant is preferably pre-gelatinized starch.

5. The dosage form of claim 4, wherein the content of rasagiline citrate is 0.74 mg to 3.63 mg, and which in addition to the rasagiline citrate, comprises mannitol, colloidal silicon dioxide, starch NF, pregelatinized starch, stearic acid, talc, hypromellose, methacrylic acid ethyl acrylate copolymer, talc extra fine, and triethyl citrate.

6. The dosage form of claim 4 or 5, wherein the core is in the form of a tablet,
wherein the content of rasagiline is 1.0 mg and the tablet comprises 45.0 mg of mannitol, or wherein the content of rasagiline is 0.5 mg and the tablet comprises 45.5 mg of mannitol, and
wherein the tablet further comprises 0.4 mg of aerosil, 5.0 mg of starch NF, 20.0 mg of pregelatinized starch, 1.5 mg of stearic acid, 1.5 mg of talc, 3.5 mg of hypromellose, 4.0 mg of methacrylic acid ethyl acrylate copolymer, 0.8 mg of triethyl citrate, 1.9 mg of talc extra fine, and 2.0 mg of a color coating agent.

7. The dosage form of any one of claims 3-6, wherein the acid resistant pharmaceutically acceptable coating comprises methacrylic acid - ethyl acrylate copolymer (1:1) and a plasticizer,
wherein the ratio of methacrylic acid - ethyl acrylate copolymer (1:1) to plasticizer is preferably between 10 to 1 and 2 to 1, more preferably about 5 to 1,
wherein the plasticizer is triethyl citrate,
wherein the acid resistant pharmaceutically acceptable coating further comprises talc,
wherein the acid resistant pharmaceutically acceptable coating is between 3% and 12% by weight of the dosage form, preferably about 8% by weight of the dosage form, or
wherein the acid resistant pharmaceutically acceptable coating comprises two coating layers, preferably the inner one of the two coating layers comprises hypromellose.

8. The dosage form of any one of claims 1-7,
which releases between 80 and 100% of rasagiline when placed in a basket apparatus in 500 mL of buffered aqueous media at a pH of 6.8 at 37°C at 75 revolutions per minute for 20 minutes,
which when ingested by a human subject achieves substantially the same MAO-B inhibition as that of the corresponding dosage of rasagiline ingested as an immediate release formulation,
which dosage form when ingested by a human subject provides an AUC value of rasagiline of 80-130%, preferably 80-125%, of that of the corresponding amount of rasagiline ingested as an immediate release formulation,
which dosage form when ingested by a human subject in a fed state provides an AUC value of rasagiline which is greater than that of the corresponding amount of rasagiline ingested as an immediate release formulation,
which dosage form when ingested by a human subject provides a Cₘₐₓ of rasagiline 80-145%, preferably 80-125%, of that of the corresponding amount of rasagiline ingested as an immediate release formulation, or
which dosage form when ingested by a human subject in a fed state provides a Cₘₐₓ of rasagiline which is greater than that of the corresponding amount of rasagiline ingested as an immediate release formulation.

9. The dosage form of any one of claims 1-8, wherein the total amount of non-polar impurities is less than 0.3 wt% relative to the amount of rasagiline, or wherein the amount of N-(2-chloroallyl)-1(R)-aminoindan in the dosage form is less than 20 ppm, preferably less than 4 ppm, relative to the amount of rasagiline.

10. Rasagiline citrate.

11. The rasagiline citrate of claim 10, which is isolated rasagiline citrate, which is solid amorphous rasagiline citrate, or which is substantially pure.

12. The rasagiline citrate of any one of claims 3, 10 and 11,
which is mono-rasagiline citrate,
which has a rasagiline content of between 42% and 52% by weight based on the total weight of the rasagiline citrate, or
which has a water content, as determined by Karl Fischer analysis, of less than 5%.

13. A composition, preferably in the form of a tablet, comprising the rasagiline citrate of any one of claims 10-12 and a carrier,
the composition wherein the rasagiline content present in the form of rasagiline citrate is more than 50% of the total rasagiline content in the composition,
the composition wherein the rasagiline citrate is mixed with a polymer, or
the composition which is a pharmaceutical composition and the carrier is a pharmaceutically acceptable carrier.

14. The composition of claim 13, further comprising rasagiline base, preferably present in an amount of less than 5% based on the total rasagiline content of the composition, or which is free of rasagiline base, or further comprising stearic acid.

15. A process for manufacture of the rasagiline citrate of any one of claims 10-12 or the composition of claim 13 or 14, comprising:
a) combining a solution of citric acid with rasagiline base to form a first mixture;
b) adding a solvent, preferably acetone, to the first mixture to form a second mixture;
c) completely removing liquid, preferably at ambient temperature and at reduced pressure, from the second mixture; and
d) recovering the rasagiline citrate or preparing the composition.

16. Use of the dosage form of any one of claims 1-9 or of the composition of claim 13 or 14, in an amount effective to treat a human subject afflicted with Parkinson's disease,
wherein the human subject optionally suffers from delayed gastric emptying, or the human subject is optionally in a fed state.
